# EUROPEAN PATENT SPECIFICATION

(11) **EP 1 479 396 B1**
(45) Date of publication and mention of the grant of the patent: **21.03.2007**
(21) Application number: 03734884.4
(22) Date of filing: 30.01.2003
(51) Int. Cl.: A61K 45/00, A61P 25/00, G01N 33/68, A61K 38/00, A61K 39/00, C12Q 1/48

(54) **AGENT FOR CONTROLLING CIRCADIAN RHYTHM DISORDER**
ARZNEIMITTEL ZUR BEHANDLUNG VON STÖRUNGEN DES ZIRKADIANEN RHYTHMUS
MEDICAMENT AMELIORANT LES TROUBLES DU RYTHME CIRCADIEN

(30) Priority: 31.01.2002 JP 2002022857
(43) Date of publication of application: 24.11.2004
(73) Proprietor: DAIICHI PHARMACEUTICAL CO., LTD., Chuo-ku, Tokyo 103-8234 (JP)
(72) Inventor: DOI, Hirofumi, c/o Celestar Lexico-Sciences, Inc., Mihama-ku, Chiba-shi, Chiba 261-8501 (JP); WADA, Naoya, c/o Daiichi Pharmaceutical Co., Ltd., Edogawa-ku, Tokyo 134-8630 (JP)
(74) Representative: Krauss, Jan
(86) International application number: PCT/JP2003/000881
(87) International publication number: WO 2003/063907

(56) References cited:
- WO-A1-00/31132
- WO-A1-99/18193
- LEE CHOOGON ET AL: "Posttranslational mechanisms regulate the mammalian circadian clock" CELL, vol. 107, no. 7, 28 December 2001 (2001-12-28), pages 855-867, XP002330612 ISSN: 0092-8674
- KOMON SANADA ET AL.: 'Mitogen-activated protein kinase phosphorylates and negatively regulates basic helix-loop-helix-PAS transcription factor BMAL1' THE JOURNAL OF BIOLOGICAL CHEMISTRY vol. 277, no. 1, 04 January 2000, pages 267 - 271, XP002967760
- KAMON SANADA ET AL.: 'Role of circadian activation of mitogen-activated protein kinase in chick pineal clock oscillation' THE JOURNAL OF NEUROSCIENCE vol. 20, no. 3, 2000, pages 986 - 991, XP002967761
- SATO HONMA ET AL.: 'Circadian oscillation of BMAL1, a partner of a mammalian clock gene clock, in rat suprachiasmatic nucleus' BIOCHEMICAL AND BIOPHYSICAL RESEARCH COMMUNICATIONS vol. 250, 1998, pages 83 - 87, XP002967762

## Description

### TECHNICAL FIELD

The present invention relates to control of circadian rhythm disorders, wherein the control is attained by inhibiting suppression of BMAL1 function, where the suppression is caused by phosphorylation of BMAL1 by c-Jun N-terminal kinase 3 (JNK3) due to the interaction between BMAL1 and JNK3.

### BACKGROUND ART

Circadian rhythm is a rhythm having a cycle that takes approximately 24 hours, and is observed in various physiological phenomena among physiological functions within a living organism, such as sleep/wakefulness, feeding, drinking, body temperature, endocrine, metabolic function or immunological functions. The biological clock that keeps this rhythm exists in all living organisms. In mammals, it has been shown that the biological clock resides in the hypothalamic suprachiasmatic nuclei of the brain.

In humans, the sympathetic nervous system is generally active during the day, which leads blood pressure, pulse and body temperature to increase and, in terms of the endocrine system, secretion of hormones from the adrenal gland, the thyroid gland and the reproductive organs to be stimulated. Conversely, production of lymphocytes and T-cells in the immune system is activated during the night. The rhythms in endocrine and metabolism produce rhythms in, for example, absorption rate and clearance time of drugs, which results in appearance of rhythms for drug sensitivity. Accordingly, abnormal circadian rhythms give rise to various disorders, such as sleep disorders (Non-Patent Reference No. 1) and psychological disorders (Non-Patent Reference No. 2), including winter depression, periodic hypertension and irregular ovulation cycles. Furthermore, links have been reported between the periodic nature of insulin secretion and diabetes (Non-Patent Reference No. 3). Meanwhile, artificial manipulation of this timing mechanism makes it possible not only to treat the disorders described above, but to eliminate jet lag syndromes resulting from flying east or west (Non-Patent Reference No. 4), and to modulate the ovulation and times of birth of livestock. Furthermore, it is possible to maximize drug efficacy while minimizing side effects, according to timing of drug administration.

Recently, a vast amount of genetic research into circadian rhythms has been conducted, giving findings of genes that are associated with the biological clocks of mammals, such as Clock, Bmall (Mop3), Period (Per2, Per3), Time (Tim), Cryptochrome (Cryl/Cry2) and casein kinase le (Non-Patent Reference No. 5 and Non-Patent Reference No. 6). Moreover, attempts have been made to elucidate a mechanism of circadian rhythm, using experimental models such as mice in which these genes have been knocked out; as a result, it has been shown that the mechanisms include transcriptional feedback loops for these genes. Concretely, BMAL1 protein and CLOCK protein form a heterodimer, which enhances the transcription of Per1 and Per2 (both of which are genes associated with the biological clock) by binding to the E-boxes (CACGTG) on their promoters. PER1 and PER2 are consequently produced and bind to PER3 (which is constantly produced), and then enter into the nucleus. Within the nucleus, PER further binds to proteins such as TIM and CRY, and suppresses the transcription of BMAL and CLOCK (Non-Patent Reference No. 7). Consequently, BMAL and CLOCK levels decrease, which suppresses the transcription of Per1 and Per2. As a result of this suppression, production of PER1 and PER2 decreases, which recovers the suppression of BMAL and CLOCK. It is believed that the circadian rhythm is established by way of such feedback loops.

Meanwhile, c-Jun-N terminal kinase (hereinafter, JNK) is a member of a MAP kinase super family. MAP kinase (MAPK) is believed to be in a major pathway among intracellular signaling pathways for controlling cell proliferation and differentiation, and works at modulating cell proliferation and differentiation downstream of the oncogene products, Ras protein and Raf-1 protein. MAPK is activated by a cascade, where MAP kinase kinase kinase (MAPKKK) (MEKK) phosphorylates MAP kinase kinase (MAPKK)(MEK)(MKK) resulting in activation thereof and the activated MEK phosphorylates MAPK. JNK is also activated by a similar cascade as described above, wherein the cascade comprises related enzymes thereof. For example, JNK3 (which is one species of JNK) is activated by a cascade, where MEKK1 phosphorylates MKK4/MKK7 resulting in activation thereof and the activated MKK4/MKK7 phosphrylates JNK3. To date, three species of JNK have been reported: JNK1, JNK2 and JNK3. Among these, JNK3 is a protein that is specifically expressed in the cerebral nervous system and the like. It has been shown that JNK3 is expressed in states of shock, such as hypoxia, and causes impaired brain function. Accordingly, to discover proteins that interact with JNK3 and to control the functions of these proteins are useful contributions to the elucidation, prevention and/or treatment of disorders caused by JNK3 or by the proteins that interact with JNK3.

The references cited in the description of this technical background are listed below.
Non-Patent Reference No. 1: *Iyaku Zasshi* (*The Journal of Pharmacology*), 1999, Vol. 122, No. 2, pp. 458-462
Non-Patent Reference No. 2: Kawakami F., *Molecular Medicine,* 1999, Vol. 36, pp. 1161-1165
Non-Patent Reference No. 3: *Shinkei Seishin Yakuri* (*Japanese Journal of Neuropsychopharmacology*), 1996, Vol. 18, No. 10, pp.703-710
Non-Patent Reference No. 4: *Rinsho Kensa* (*Journal of Medical Technology*), 2001, Vol. 45, No. 6, pp.636-639
Non-Patent Reference No. 5: King D.P. *et al.*, *Annual Review of Neuroscience,* 2000, Vol. 23, pp.713-742
Non-Patent Reference No. 6: Maureen K.B. *et al., Cell,* 2000, Vol. 103, pp.1009-1017
Non-Patent Reference No. 7: Lee C. *et al., Molecular and Cellular Biology,* 1999, Vol. 19, pp.5316-5325

### DISCLOSURE OF THE INVENTION

As part of the present invention, various studies have been undertaken with the aim of discovering proteins that interact with JNK3 and providing means for controlling disorders caused by changes in the functions of these proteins, where the changes arise from the interaction between these proteins and JNK3. As a result, the interaction of JNK3 with BMAL1 (which is a transcription factor involved in the circadian rhythm) has been predicted *in silico* and then demonstrated experimentally. Furthermore, it was discovered that, as a result of this interaction, BMAL1 is phosphorylated by JNK3 resulting in suppression of the function thereof; consequently the present invention was completed.

That is to say, an aspect of the present invention is an agent for controlling a circadian rhythm disorder, wherein the agent is characterized by inhibiting the phosphorylation of BMAL1 by c-Jun N-terminal kinase 3.

Furthermore, an aspect of the present invention is an agent for controlling a circadian rhythm disorder, wherein the agent is characterized by comprising an effective dose of a c-Jun N-terminal kinase 3 (JNK3) expression inhibitor and/or its function inhibitor, and by inhibiting the phosphorylation of BMAL1 by JNK3.

In addition, an aspect of the present invention is a method for controlling a circadian rhythm disorder, wherein the method is characterized by inhibiting the phosphorylation of BMAL1 by c-Jun N-terminal kinase 3.

In addition, an aspect of the present invention is a method for controlling a circadian rhythm disorder, wherein the method is characterized by inhibiting the phosphorylation of BMAL1 by c-Jun N-terminal kinase 3 (JNK3), by means of inhibiting expression of JNK3 and/or inhibiting the function of JNK3.

Furthermore, an aspect of the present invention is an agent for treating and/or preventing a disease caused by a circadian rhythm disorder, wherein the agent is characterized by inhibiting the phosphorylation of BMAL1 by c-Jun N-terminal kinase 3.

In addition, an aspect of the present invention is a method for treating and/or preventing a disease caused by a circadian rhythm disorder, wherein the method is characterized by inhibiting the phosphorylation of BMAL1 by c-Jun N-terminal kinase 3.

In addition, an aspect of the present invention is a method for identifying a compound that inhibits the interaction between c-Jun N-terminal kinase 3 (JNK3) and BMAL1, wherein the method includes contacting a compound with JNK3 and/or BMAL1 under conditions allowing for the interaction of the compound with JNK3 and/or BMAL1, and determining whether the compound inhibits the interaction between JNK3 and BMAL1 by using a system that uses a signal and/or a marker generated by the interaction between JNK3 and BMAL1 to detect presence or absence or change of the signal and/or the marker.

Furthermore, an aspect of the present invention is a method for identifying a compound that inhibits the phosphorylation of BMAL1 by c-Jun N-terminal kinase 3 (JNK3), wherein the method includes contacting a compound with JNK3 and/or BMAL1, and determining whether the compound inhibits the phosphorylation of BMAL1 by JNK3, by using a system that uses a signal and/or a marker capable of detecting the phosphorylation of BMAL1 to detect presence or absence or change of this signal and/or marker.

In addition, an aspect of the present invention is a compound that inhibits the interaction between c-Jun N-terminal kinase 3 and BMAL1.

Furthermore, an aspect of the present invention is a compound that inhibits the phosphorylation of BMAL1 by c-Jun N-tenninal kinase 3.

In addition, an aspect of the present invention is an agent for inhibiting the interaction between c-Jun N-terminal kinase 3 and BMAL1.

In addition, an aspect of the present invention is an agent for inhibiting the phosphorylation of BNML1 by c-Jun N-terminal kinase 3.

In addition, an aspect of the present invention is a method for recovering the suppressed transcriptional activity of a complex comprising BMAL1 and CLOCK, wherein the method is characterized by inhibiting the expression of c-Jun N-terminal kinase 3 and/or inhibiting the function of the same.

Furthermore, an aspect of the present invention is a reagent kit for use in said identification method, wherein the kit contains at least: c-Jun N-tenninal kinase 3 (JNK3) and/or BMAL1, or a polynucleotide encoding JNK3 and/or a polynucleotide encoding BMAL1, or a vector comprising a polynucleotide encoding JNK3 and/or a vector comprising a polynucleotide encoding BMAL1.

### BRIEF DESCRIPTION OF THE DRAWINGS

Figure 1 illustrates the result of *in silico* prediction of interaction between JNK3 and BMAL1. Local alignment between JNK3 and BMAL1 was conducted, and regions with high scores were shown. The upper and lower rows indicate sequences present in JNK3 and sequences present in BMAL1, respectively.

Figure 2 illustrates the result of *in silico* prediction of interaction between JNK3 and BMAL2. Local alignment between JNK3 and BMAL2 was conducted, and regions with high scores were shown. The upper and lower rows indicate sequences present in JNK3 and sequences present in BMAL2, respectively.

Figure 3A shows the results (arrowheads) of detection of each purified glutathion S-transferase (GST) fusion protein (lane 1: GST-BMAL1, lane 2: GST, lane 3: GST-c-Jun (1-79)) by means of SDS-PAGE followed by staining with Coomassie Brilliant Blue. Lane M is a molecular weight marker.

Figure 3B shows that BMAL1 was phosphorylated *in vitro* by JNK3. GST-BMAL1 (lane 2) and GST-c-Jun (1-79) (lane 3) were phosphorylated by JNK3, whereas GST (lane 1) was not phosphorylated. The arrowheads indicate the phosphorylated GST-BMAL1 or the phosphorylated GST-c-Jun (1-79). The values shown on the left-hand side of the figure are the molecular weights of the molecular weight markers.

Figure 4 shows that BMAL1 was phosphorylated by JNK3 in a dose-dependent manner. Lane 1, lane 2, lane 3 and lane 4 show the phosphorylation of BMAL1 when JNK3 was added at 0 ng, 14 ng, 28 ng and 70 ng, respectively. The arrowhead indicates the phosphorylated GST-BMAL1. The numbers on the left-hand side of the figure are the molecular weights of the molecular weight markers.

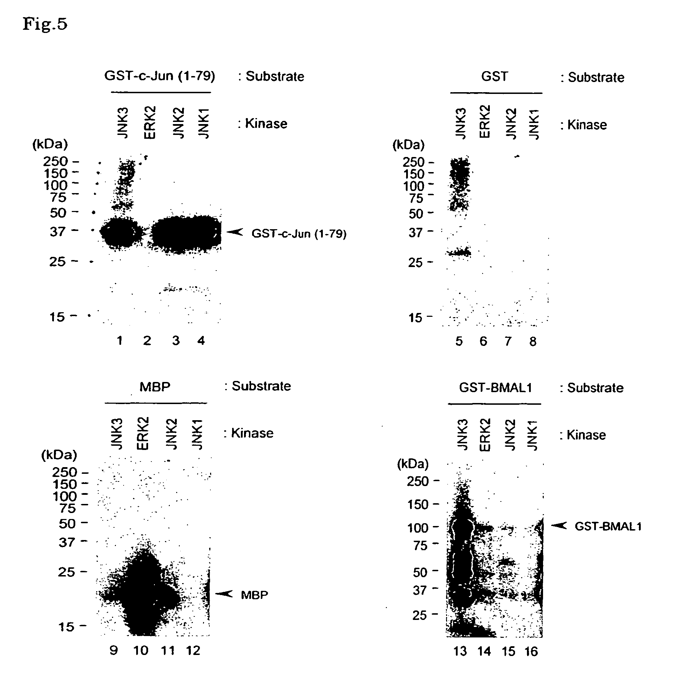

Figure 5 shows that BMAL1 was phosphorylated by JNK3, whereas it was not phosphorylated by ERK2, JNK1 or JNK2. In the phosphorylation reaction, GST-c-Jun (1-79) (lanes 1-4), GST (lanes 5-8), myelin basic protein (MBP) (lanes 9-12) and GST-BMAL1 (lanes 13-16) were used as substrates, while JNK3 (lanes 1, 5, 9, and 13), ERK2 (lanes 2, 6, 10, and 14), JNK2 (lanes 3, 7, 11, and 15) and JNK1 (lanes 4, 8, 12, and 16) were used as kinases. Each phosphorylated protein is indicated by an arrowhead. The numbers on the left-hand side of the figure are the molecular weights of the molecular weight markers.

Figure 6 shows that transcriptional activity was confirmed when both proteins of BMAL1 and CLOCK were expressed in a reporter assay. Transcriptional activity was measured by detecting luciferase activity.

Figure 7 shows that transcriptional activity resulting from the coexpression of BMAL1 and CLOCK was suppressed when JNK3 was activated by expression of an active form of MEKK1. Transcriptional activity was measured by detecting luciferase activity in a reporter assay.

Figure 8 shows that transcriptional activity resulting from the coexpression of BMAL1 and CLOCK was suppressed when JNK3 was activated by expression of an active form of MKK7. Transcriptional activity was measured by detecting luciferase activity in the reporter assay.

Figure 9A shows that the transcriptional activity resulting from the coexpression of BMAL1 and CLOCK was suppressed by the activation of JNK3 by an active form of MEKK1, and the suppression was not observed when the action of JNK3 was inhibited by coexpression of the JNK binding domain (JBD) of JIP1.

Figure 9B shows that the transcriptional activity resulting from the coexpression of BMAL1 and CLOCK was suppressed by the activation of JNK3 by an active form of MEKK 1, and the suppression was not observed when the action of JNK3 was inhibited by coexpression of the JNK binding domain (JBD) of JSAP1.

### DETAILED DESCRIPTION OF THE INVENTION

The present invention claims priority from Japanese patent application No. 2002-022857.

Technical and scientific terms used in the present specification, unless separately defined, have the meanings that are normally understood by those skilled in the art. In the present specification, reference is made to a variety of methods known to those skilled in the art.

Hereinafter, a mode of embodiment of the present invention may be described in more detail. The following detailed description is illustrative and merely explanatory, and does not limit the present invention in any way.

### Interaction of BMAL1 with JNK3 and phosphorylation of BMAL1 by an active form of JNK3

In the present invention, prediction of the proteins that interact with JNK3 was conducted according to the method set forth in the International Patent Publication No: WO 01/67299; as a result, BMAL1 was discovered to be such a protein. Furthermore, it was discovered by experiment that: BMAL1 is phosphorylated by an active form of JNK3; and the phosphorylation of BMAL1 leads to suppression of transcriptional activity of the complex comprising BMAL1 and CLOCK (hereinafter also referred to as BMAL1/CLOCK complex).

It has been reported that BMAL1 is phosphorylated by MAPK1 (which is a member of MAPK) resulting in suppression of the function thereof (Sanada K. et al, *Journal of Biological Chemistry,* 2002, Vol. 227, pp. 267-271). However, the present invention makes it clear that the phosphorylation activity of JNK3 on BMAL1 is more specific than that of MAPK1. Furthermore, suppression of the transcriptional activity of the BMAL1/CLOCK complex (where the suppression results from activation of MEKK1 that is a factor associated with the activation of both MAPK 1 and JNK3) is almost entirely recovered by peptides known to have a dominant-negative effect on JNK3 activity, such as JIP-1 partial peptides. These findings in the present invention revealed that JNK3 is both more specific and stronger than MAPK1 in terms of the function-suppressive effect through phosphorylation of BMAL1.

### Agents and methods for preventing and/or treating diseases caused by circadian rhythm disorders, by means of inhibiting the interaction between JNK3 and BMAL1 or inhibiting the phosphorylation of BMAL1 by JNK3

BMAL1 is a transcription factor involved in modulation of the circadian rhythm. It is known that reduced transcriptional activity of the BMAL1/CLOCK complex is accompanied by decreased expression of Per and Cry which are circadian rhythm regulators (Gekakis N. *et al.*, *Science,* 1998, Vol. 280, pp.1564-1569), and that the decreased expression of Per and Cry leads to circadian rhythm disorders (Non-Patent Reference No. 6).

Meanwhile, the MAPK cascade is reported to be associated with resetting the circadian rhythm (Akashi Makoto *et al.*, *Cell Technology,* 2001, Vol. 20, pp. 822-827; Okano Toshiyuki *et al.*, *Cell Technology,* 2001, Vol. 20, pp. 837-842). Here, the expression "resetting the circadian rhythm" means synchronizing intrinsic rhythm mechanism in living organisms with external time, by means of external environmental factors such as light and temperature. Specifically, in humans, the intrinsic rhythm mechanism has a period of 25 hours, which is longer than an actual day, though this is synchronized with external time of 24 hours as a result of exposure to light and the like.

Based on these reports and the findings that have been revealed in the present invention, the inventors believe that: while the circadian rhythm is being modulated by MAPK, if JNK3 is activated as a result of stress or the like, a phosphorylation reaction of BMAL1 (that is stronger and more specific than that by MAPK1) takes place; subsequently, the considerable suppression of transcription of circadian rhythm regulators occurs, and consequently, circadian rhythm disorders happen.

Therefore, it is possible to control circadian rhythm disorders by means of inhibiting the phosphorylation of BMAL1 by JNK3.

Accordingly, the present invention is capable of providing a method for controlling a circadian rhythm disorder and a method for treating and/or preventing a disease caused by this disorder, as well as an agent for controlling a circadian rhythm disorder and an agent for treating and/or preventing a disease caused by this disorder, wherein the method and the agent are characterized by inhibiting the phosphorylation of BMAL1 by JNK3.

The disease caused by a circadian rhythm disorder is exemplified by sleep/wakefulness rhythm disorders, cyclic/recurrent disorders, and the like; however, it is not limited thereto. Examples of sleep/wakefulness rhythm disorders include delayed sleep phase syndrome and non-24-hour sleep patterns. Examples of cyclical/recurrent disorders include endogenous manic depressive psychosis, seasonal affective disorder, cyclic catatonia, cyclic high blood pressure, cyclic ulcers, irregular ovulation cycles, and diabetes caused by cyclic abnormalities in insulin secretion.

Furthermore, a circadian rhythm disorder is thought to be associated with nocturnal wandering in cerebrovascular dementia and Alzheimer's dementia. In addition, stress, chronic fatigue, lowered resistance to infection, jet lag, and the like can be ascribed to a circadian rhythm disorder. Furthermore, the inventors believe that a circadian rhythm disorder may be associated with drug efficacy and the incidence of side effects when administering the drug.

### Method of identifying a compound for inhibiting the interaction between JNK3 and BMAL1 or for inhibiting the phosphorylation of BMAL1 by JNK3

The present invention provides a method for identifying a compound for inhibiting the interaction between JNK3 and BMAL1, where the interaction is exemplified by phosphorylation of BMAL1 by JNK3. The method can be established using systems for pharmaceutical screening that are well-known in the art. JNK3 and BMAL1 used for identifying the compound can be in cells in which these are expressed by means of genetic engineering techniques, the products of cell-free synthesis systems, the chemical synthesis products, or those obtained from the cells or from any biological samples. These can be subsequently further purified for use. Furthermore, as long as the interaction between JNK3 and BMAL1 and the function of either protein is not disturbed, JNK3 and BMAL1 can be those to which a different type of protein or peptide (for example, β-gatactosidase, an Fc fragment of an immunoglobulin such as immunoglobulin G (IgG), His-tag, Myc-tag, Flag-tag and the like) are ligated at the N-terminus or the C-terminus thereof, directly or indirectly via a linker peptide. Ligation of these proteins or peptides can be performed by using well-known methods such as genetic engineering techniques. Examples of compounds to be screened include compounds derived from chemical libraries and natural substances, as well as compounds obtained by drug design based on the three-dimensional structures of JNK3 and BMAL1.

For example, compounds that inhibit the interaction between JNK3 and BMAL1 can be identified by selecting conditions that allow for interactions of a test compound with JNK3 and/or BMAL1, bringing JNK3 and/or BMAL1 to contact with the compound under the conditions, employing an assay system using a signal and/or a marker that makes it possible to detect the interaction between JNK3 and BMAL1, and detecting the presence, the absence, or the change in the signal and/or the marker. The term "signal" as used herein refers to a substance that can be detected directly by itself based on the physical properties or chemical properties thereof. The term "marker" refers to a substance that can be detected indirectly when the physical properties or biological properties thereof are used as an indicator. In terms of signals, luciferase, green fluorescent protein (GFP), radioactive isotopes and the like can be used; in terms of markers, reporter genes such as the chloramphenicol acetyl transferase (CAT) gene, or detectable tags such as the 6xHis-tag can be used. However, all substances that are well-known can be used. Methods for detecting these signals and markers are known to persons skilled in the art.

Specifically, compounds that inhibit the phosphrylation of BMAL1 by JNK3 can be identified by bringing JNK3 and/or BMAL1 to contact with the compound, employing an assay system using a signal and/or a marker that makes it possible to detect the phosphorylation of BMAL1, and detecting the presence, the absence, or the change in the signal and/or the marker. It is preferable that JNK3 be in an active form at this time. If JNK3 is in an inactive form, an enzyme that activates JNK3 but that does not phosphorylate BMAL1 can be used together with JNK3. Detection of the phosphorylation of BMAL1 can be carried out using a protein phosphorylation analysis and quantitative method for measuring phosphorylated protein, where the method is well-known in the art. For example, in a simple method, detection of the phosphorylation of BMAL1 can be performed quantitatively by bringing JNK3 to react with BMAL1 in the presence of [γ-³²P] ATP, separating the proteins using SDS-PAGE after the reaction, detecting the bands showing the proteins by way of staining, and then, measuring the radioactivity of the band corresponding to the phosphorylated BMAL1. The reaction described above can be performed in the presence or absence of a given compound so as to compare the results, which makes it possible to determine whether the compound in question inhibits the phosphorylation of BMAL1 by JNK3.

Alternatively, compounds that inhibit the interaction between JNK3 and BMAL1 can be identified by using cells in which JNK3 and BMAL1 have been expressed, bringing the cells to contact with the compound, employing an assay system using a signal and/or a marker that makes it possible to detect the interaction between JNK3 and BMAL1, and detecting the presence, the absence, or the change in the signal and/or the marker. The interaction between JNK3 and BMAL1 can, for example, be detected by measuring the phosphorylation of BMAL1. Otherwise, this can be detected by coexpressing CLOCK in the cell, introducing a reporter gene for the purpose of detecting the transcriptional activity of the BMAL1/CLOCK complex, and measuring the reporter activity. A firefly luciferase reporter plasmid having three E-boxes that have been introduced upstream of the SV40 promoter is used as the reporter gene in an example of the present invention. However, the reporter gene is not limited to this and may be freely chosen, as long as it allows for detection of the transcriptional activity of the BMAL1/CLOCK complex.

Identification methods using cells as described above may be used in combination with *in vitro* identification methods such as those described above. Compounds (which inhibit phosphorylation of BMAL1 by JNK3) obtained by *in vitro* identification methods can be subjected to further experimentation with identification methods using cells, in order to select the compounds that inhibit, in the cells, phosphorylation of BMAL1 by JNK3 and thereby achieve suppression of BMAL1 function as a result of the phosphorylation.

### Agents for inhibiting the interaction between JNK3 and BMAL1 or for inhibiting the phosphorylation of BMAL1 by JNK3, and pharmaceutical compositions

Compounds obtained by the identification method described above can be used as agents for inhibiting the interaction between JNK3 and BMAL1, such as agents for inhibiting the phosphorylation of BMAL1 by JNK3. Such compounds can be exemplified by peptides and oligopeptides comprising the amino acid sequences of sites at which the two proteins interact. Such peptides or oligopeptides can be identified by firstly designing them based on the amino acid sequences of JNK3 or BMAL1, synthesizing them by peptide synthesis methods well-known in the art, and examining whether they can inhibit phosphorylation of BMAL1 by JNK3 in the identification method described above. The compounds described above are also exemplified by an antibody that inhibits the interaction between JNK3 and BMAL1. The antibody can, for example, be produced using the peptides or the oligopeptides (comprising the amino acid sequences of sites at which the two proteins interact) as antigens, by using antibody preparation methods well-known in the art.

Alternatively, compounds that inhibit the expression and/or function of JNK3 are also included within the scope of the present invention, since such compounds are, in effect, able to inhibit phosphorylation of BMAL1 by JNK3. Compounds that inhibit the expression of JNK3 can be identified using screening systems well-known in the art for obtaining inhibitors of protein expression. Compounds that inhibit the expression of JNK3 can be exemplified by antisense oligonucleotides of JNK3 gene. The antisense oligonucleotides can be obtained from oligonucleotides that are designed based on the base sequence of the JNK3 gene, by selecting ones that specifically inhibit the expression of the JNK3 gene using a JNK3 gene expression system. Furthermore, compounds that inhibit the function of JNK3 can be obtained by using JNK3 to select substances that inhibit a function of the same (such as kinase activity or the interaction with BMAL1), using the identification method described above. Concrete examples of such compounds include JNK binding domain (Jun kinase binding domain: hereinafter, JBD)-partial peptides of JIP1, JSAP1 and the like. JIP1 and JSAP1 are known as scaffold proteins for the JNK cascade, and the JBD-partial peptides thereof are known to have a dominant negative effect on JNK3 activity. The present invention reveals that JBD-partial peptides almost entirely recover the suppressed transcriptional activity of BMAL1/CLOCK complex, where the suppression results from activation of MEKK1 that is a factor involved in the activation of both MAPK1 and JNK3. An agent for recovering the suppressed transcriptional activity of BMAL1/CLOCK complex is included within the scope of the present invention, wherein the agent comprises the aforementioned agent for inhibiting the expression of JNK3 and/or the aforementioned agent for inhibiting the function of JNK3.

Compounds selected in this manner, agents for inhibiting the interaction between JNK3 and BMAL1, agents for inhibiting the phosphorylation of BMAL1 by JNK3, and agents for recovering the suppressed transcriptional activity of BMAL1/CLOCK, can be used for agents and methods for controlling circadian rhythm disorders by using them alone or in combination. Furthermore, these compounds, inhibitors, and agents for recovering the suppressed transcriptional activity can be used as reagents. These reagents can, for example, be used in the study of circadian rhythms and disorders thereof.

Compounds selected in this manner, agents for inhibiting the interaction between JNK3 and BMAL1, agents for inhibiting the phosphorylation of BMAL1 by JNK3, and agents for recovering the suppressed transcriptional activity of BMAL1/CLOCK, can be formulated as pharmaceutical compositions by way of further selection with consideration for the balance between biological effectiveness and toxicity. These compounds, inhibitors, and agents for recovering the suppressed transcriptional activity can each be used singularly or in combination.

As circadian rhythm disorders may arise when JNK3 and BMAL1 interact and BMAL1 is phosphorylated by JNK3, the pharmaceutical compositions described above are useful in controlling circadian rhythm disorders. Furthermore, the pharmaceutical compositions according to the present invention can be used in the treatment and/or prevention of diseases caused by circadian rhythm disorders. The diseases caused by circadian rhythm disorders are exemplified by sleep/wakefulness rhythm disorders, cyclic/recurrent disorders, and the like; however, it is not limited thereto. Examples of sleep/wakefulness rhythm disorders include delayed sleep phase syndrome and non-24-hour sleep patterns. Examples of cyclical/recurrent disorders include endogenous manic depressive psychosis, seasonal affective disorder, cyclic catatonia, cyclic high blood pressure, cyclic ulcers, irregular ovulation cycles, and diabetes caused by cyclic abnormalities in insulin secretion. Furthermore, circadian rhythm disorders are thought to be associated with nocturnal wandering in cerebrovascular dementia and Alzheimer's dementia. In addition, stress, chronic fatigue, lowered resistance to infection, jet lag, and the like can be ascribed to circadian rhythm disorders. Accordingly, the pharmaceutical compositions can be used for these diseases. Furthermore, as circadian rhythm disorders may be associated with drug efficacy and the incidence of side effects when administering a drug, the pharmaceutical compositions can be administered in combination with or separately from other medicaments, so as to increase the drug effect and/or diminish the side effects of these other medicaments.

In terms of the formulation of the agents for inhibiting the interaction between JNK3 and BMAL1, the agents for inhibiting the phosphorylation of BMAL1 by JNK3, the agents for recovering the suppressed transcriptional activity of BMAL1/CLOCK complex, the agents for controlling circadian rhythm, and the pharmaceutical compositions, it is preferable that these be formulated in combination with suitable pharmaceutical carriers. Such formulation comprises a therapeutically effective dose of the aforementioned compound, inhibitor, or agent for recovering the suppressed transcriptional activity, controlling agent, and/or pharmaceutical composition, and a pharmaceutically acceptable carrier or vehicle. Examples of such a carrier include: physiological saline solution, buffered physiological saline solution, dextrose, water, glycerol, ethanol, and mixtures thereof; however, they are not limited thereto. The formulation can be selected according to the administration route, and formulations are well-known to those skilled in the art. The aforementioned inhibitors, agents for recovering the suppressed transcriptional activity, controlling agents, and/or pharmaceutical compositions can be used alone or together with other compounds or medicaments that are therapeutically effective.

In terms of the mode of administration for the agents for inhibiting the interaction between JNK3 and BMAL1, the agents for inhibiting the phosphorylation of BMAL1 by JNK3, the agents for recovering the suppressed transcriptional activity of BMAL1/CLOCK complex, the agents for controlling circadian rhythm, and the pharmaceutical compositions, these can be administered systemically or locally. One preferred mode of systemic administration is injection, such as intravenous injection. Other injection routes, such as subcutaneous, intramuscular or intraperitoneal injection, can also be used. Another mode of administration can be peroral administration, if an enteric formulation or capsule formulation can be suitably formulated. In addition, permucosal administration or percutaneous administration using a permeating agent such as bile salt, fusidic acid or other surfactants can also be used. Topical administration can be in the form of plaster, paste, gel, etc.

The dosage range required can be determined according to the effectiveness of the inhibitors, the agents for recovering the suppressed transcriptional activity, the controlling agents, and the pharmaceutical compositions; the administration route; the characteristics of the formulation; the nature of the symptoms to be treated; and the judgment of the doctor in attendance. Specifically, an adequate dose may, for example, fall within the range of 0.1 µg to 100 µg per 1 kg of body weight of subject. However, the doses can be modified by means of common conventional experiments for optimization, which are well known in the art.

In terms of pharmaceutical preparation, well-known means therefor can be introduced according to the physical properties of the various targets such as peptides, proteins, oligonucleotides, compounds, and the like. Specifically, methods for pharmaceutical preparation such as powdered drugs, pills, tablets, capsules, aqueous solutions, ethanol solutions, liposome preparations, fat emulsions, or clathrates (such as those of cyclodextrin) can be used.

Powdered drugs, pills, capsules and tablets can be prepared using excipients such as lactose, glucose, sucrose and mannitol; disintegrants such as starch and sodium alginate; lubricants such as magnesium stearate and talc; binders such as polyvinyl alcohol, hydroxypropyl cellulose and gelatin; surfactants such as fatty acid esters; and plasticizers such as glycerin. For preparation of a tablet or a capsule, a solid pharmaceutical carrier is used.

A suspension can be prepared using water; sugar such as sucrose, sorbitol and fructose; glycols such as polyethylene glycol (PEG); and oils.

Injectable solutions can be prepared using a saline solution, a glucose solution, and a carrier comprising a mixture of salt water and glucose solution.

Inclusion into liposomes can be performed, for instance, by adding a solution that is prepared by dissolving the substance of interest in a solvent (such as ethanol) to a solution that is prepared by dissolving phospholipids in an organic solvent (such as chloroform); then removing the solvent by evaporation; subsequently adding a phosphate-buffered solution thereto followed by agitating and sonicating thereof; and finally centrifuging thereof to obtain the supernatant and filtrating it for recovery.

Fat emulsions can, for example, be prepared by mixing the substance of interest, oil ingredients (vegetable oils such as bean oil, sesame oil and olive oil as well as MCT and the like), emulsifying agents (such as phospholipids) and the like, followed by heating to obtain a solution; then adding the necessary amount of water;and emulsifying/homogenizing with a homogenizer (for example, high-pressure-spray type, sonicating type or the like). Furthermore, this can also be lyophilized. Moreover, when emulsifying fat, an auxiliary emulsifier may be added. Auxiliary emulsifiers are exemplified by glycerin and sugars (such as glucose, sorbitol, fructose, and the like).

Cyclodextrin clathrates can, for example, be prepared by adding a solution that is prepared by dissolving cyclodextrin in water or the like by heating to a solution that is prepared by dissolving the substance of interest in a solvent (such as ethanol); then cooling and filtrating the precipitate; and dry-sterilizing. At this point, the cyclodextrin to be used can be suitably selected from cyclodextrins with different void diameters (α, β and γ types) according to the size of the substance.

### Reagent kits

The present invention provides a reagent kit that comprises at least BMAL1 and JNK3, or a polynucleotide encoding JNK3 and a polynucleotide encoding BMAL1, or a vector containing a polynucleotide encoding JNK3 and a vector containing a polynucleotide encoding BMAL1. This reagent kit can be used in the identification method described above. JNK3 and BMAL1 can be in cells in which these are expressed by means of genetic engineering techniques, the products of cell-free synthesis systems, the chemical synthesis products, or those obtained from the cells or from any biological samples. These can be subsequently further purified for use. Furthermore, as long as the interaction between JNK3 and BMAL1 and the function of either protein is not disturbed, JNK3 and BMAL1 can be those to which a different type of protein or peptide (for example, β-galactosidase, an Fc fragment of an immunoglobulin such as immunoglobulin G (IgG), His-tag, Myc-tag, Flag-tag and the like) is ligated at the N-terminus or the C-terminus thereof, directly or indirectly via a linker peptide. The polynucleotide encoding JNK3 or BMAL1 can be prepared from human cDNA libraries by means of genetic engineering techniques that are well known in the art. The vector that contains the polynucleotide encoding JNK3 or BMAL1 can be obtained by introducing the polynucleotide into suitable expression vector DNA (such as a bacterial plasmid derived vector), by means of genetic engineering techniques that are well-known in the art. The reagent kit can further contain substances necessary for the identification method described above, such as signals and/or markers for detecting interactions between JNK3 and BMAL1 (for example, phosphorylation of BMAL1 by JNK3, or suppression of the function of BMAL1 by JNK3) and a buffer. In terms of the preparation thereof, it is sufficient to use a well-known means for the preparation suitable for each substance to be used.

### EXAMPLES

The present invention may be described more concretely in the following examples but the present invention is not limited to these examples.

### Example 1

### In silico search for proteins that interact with JNK3

Prediction of the proteins that interact with JNK3 was conducted according to the method set forth in the International Patent Publication No: WO 01/67299. First, the amino acid sequence of JNK3 was decomposed into oligopeptides having a pre-determined length to search in a database for proteins having the amino acid sequence of each of the oligopeptides, or having the homologous amino acid sequences to these amino acid sequences. Next, the local alignment between proteins obtained and JNK3 was conducted to predict that the proteins having a high local alignment score might be those interacting with JNK3. The criteria for the local alignment score was the same as that in the method described in the International Patent Publication No: WO 01/67299, which is to say no less than 25.0. Furthermore, JNK3 is a protein that is specifically expressed in the cerebral nervous system and is known to impair brain function when expressed in states of shock such as hypoxia. Accordingly, candidate proteins that may interact with JNK3 were narrowed down to proteins known to be expressed in the brain and having important functions.

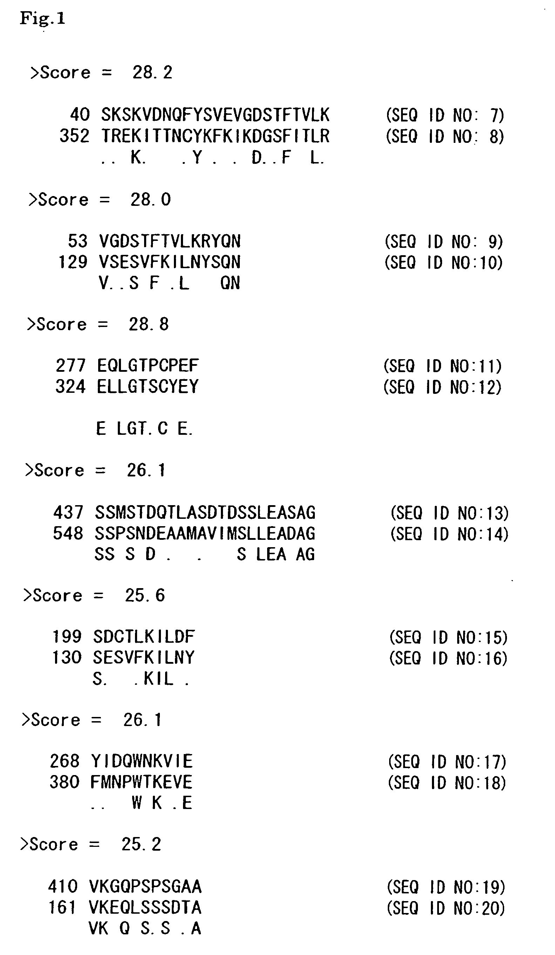

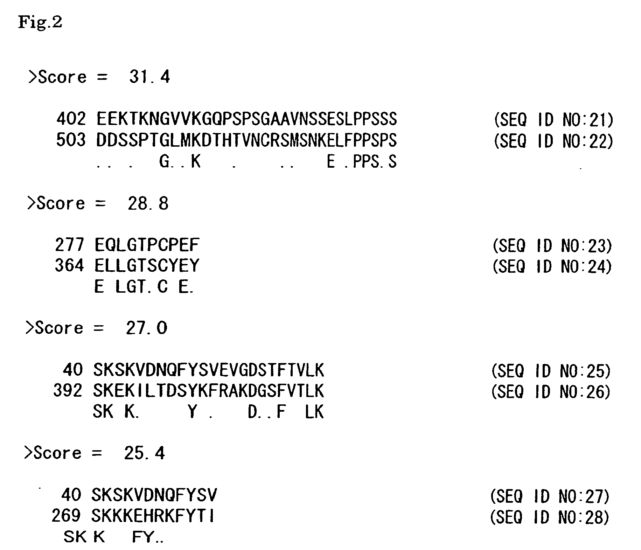

Consequently, it was found that the peptide KVKEQL (SEQ ID NO: 3), which has homology to the oligopeptide KVIEQL (SEQ ID NO: 2) comprising six amino acid residues derived from JNK3, is present in the amino acid sequence of the protein BMAL1 that is implicated in the circadian rhythm. In addition, it was also found that the oligopeptide KVKEQL (SEQ ID NO: 3) was present in the amino acid sequence of the protein BMAL2 that is homologous to BMAL1. Figure 1 and Figure 2 show the results of local alignment between JNK3 and BMAL1 and between JNK3 and BMAL2, respectively. Between JNK3 and BMAL1, seven fragments that have local alignment scores of 25.0 or greater were found; between JNK3 and BMAL2, four fragments were found. Consequently, it was predicted that BMAL1 is a protein that interacted with JNK3 more strongly than BMAL2.

### Analysis of interaction between JNK3 and BMAL1 and phosphorylation of BMAL1

In order to experimentally determine whether BMAL1 was a substrate for JNK3, *in vitro* phosphorylation experiments were performed using active JNK3.

### Materials

An active form of human JNK3 was expressed as an N-terminal His-tagged protein (His-JNK3) in Sf9 cells, and then purified using Invitrogen Probond Resin. Consequently, human JNK3 (JNK3 α1) cDNA (which was obtained by a reverse transcription polymerase chain reaction (RT-PCR) using a human hippocampus cDNA library as a template) was inserted into pFASTBAC HT (Invitrogen) to construct a recombinant baculovirus for His-JNK3 expression according to the manufacturer's instructions. Sf9 cells were infected with the recombinant virus that had been constructed, and then the infected cells were solubilized with Lysis buffer (20 mM Tris-HCl, pH 7.6/0.15 M NaCl/1% NP-40/1 mM Na₃VO₄/2.5 mM Na-pyrophosphate/1 mM β-glycerophosphate/I mM benzamidin/10 units/ml aprotinin/protease inhibitor cocktail), followed by centrifugation to collect the supernatant. The supernatant was applied to a Probond Resin (Invitrogen) column and rinsed with a buffer A (20 mM Tris-HCl, pH 7.6/0.15 M NaCl/1 mM Na₃VO₄/1mM benzamidin/10 units/ml aprotinin); the target protein was obtained by linear gradient elution with buffer A containing 0-0.2 M of imidazole. The fraction containing His-JNK3 was stored at -80°C until the time of use.

c-Jun (1-79) (the N-terminal 79 amino acid region of c-Jun, including the site that is phosphorylated by JNK) was expressed in *E. coli* as an N-terminal GST fusion protein (hereinafter, GST-c-Jun (1-79)), and then purified with Glutathione Sepharose 4B (Amersham Pharmacia Biotech); it was used as a positive control for measuring the kinase activity of JNK3.

Human BMAL1 was expressed in *E. coli* as an N-terminal GST fusion protein (hereinafter, GST-BMAL1), and then purified with Glutathione Sepharose 4B (Amersham Pharmacia Biotech). Concretely, first, an open reading frame (ORF) region of BMAL1 (which had been amplified by means of PCR from a C-terminal V5/His-tagged human BMAL1 expression plasmid, pcDNA 3.1-BMAL1/V5-His (Invitrogen)) was inserted into pGEX-4T (Amersham Pharmacia Biotech) to construct pGEX-BMAL1 that is a GST-BMAL1 expression vector for *E. coli.* After culturing *E. coli* strain BL21, harboring pGEX-BMAL1, at 37°C in 400 ml of LB culture medium containing 100 µg/ml of ampicillin until the OD₆₀₀ reached approximately 1.5, isopropyl-1-thio-β-D-galactoside (IPTG) was added to a final concentration of 0.3 mM; after further culturing at 25°C for six hours, the bacteria were collected. After washing the bacteria with 40 ml of 1% sarkosyl/l mM ethylenediamine tetraacetate (EDTA)/phosphate-buffered physiological saline (PBS)(pH 7.4), the bacteria were suspended in 40 ml of TGEDS buffer (50 mM Tris-HCl, pH 8.0/0.2 mM EDTA/1 mM dithiothreitol (DTT)/150 mM NaCl/10% glycerol) containing a proteinase inhibitor cocktail, and sonicated. Then, Triton X-100 was added to a final concentration of 1.0%. This was left to stand for 15 minutes on ice, and then centrifuged at 20,000 g for 30 minutes to collect the supernatant. The supernatant was added to Glutathione Sepharose 4B in the amount of 2 ml (which had been pre-equilibrated with TGEDS buffer) and mixed by inverting tube for 1.5 hours at 4°C to make the target protein adhere to the resin. After washing the resin three times with a 10-fold volume of TGEDS/0.1% Triton X-100, this was packed into a column and the protein of interest was eluted with TGEDS/0.1% Triton X-100 containing 10mM of Glutathione. The eluate was fractionated in 0.5 ml aliquots; the fraction containing the target protein was identified by SDS-PAGE, and collected. The collected eluate was dialyzed overnight with TGEDS/0.1% Triton X-100, and concentrated using a Microcon YM-30 (Millipore). Thereafter this was stored at -80°C until the time of use.

### Method

The *in vitro* kinase assay was performed by incubating 1 µg of each aforementioned GST fusion proteins (GST-BMAL1, GST-c-Jun (1-79) or GST) and an active form of JNK3 (70 ng) for 30 minutes at 30°C in 20 µl of kination buffer (25 mM Tris-HCl, pH7.5/5 mM β-glycerophosphate/2 mM DTT/0.1 mM Na₃VO₄/10 mM MgCl₂/10 µM ATP) containing 5 µCi [γ-³²P] adenosine triphosphate (ATP) (3000 Ci/mmol, NEN). After the reaction, 20 µl of 2 × SDS sample buffer (4% SDS/125 mM Tris-HCl, pH 6.8/20% glycerol/0.01% bromophenol blue (BPB)/10% β-mercaptoethanol) were added and treated for 5 minutes at 100°C, then the proteins were separated by 5%-20% SDS-PAGE. Next, phosphorylated proteins were detected by autoradiography with BAS 2000 (Fuji Film). In addition, the degree of migration of the target protein was verified by Coomassie Brilliant Blue (CBB) stain after separating 1 µg of each GST fusion proteins (that were used) by 5%-20% SDS-PAGE. Furthermore, in order to test for dose dependency of JNK3 in the BMAL1 phosphorylation reaction, detection of phosphorylated protein was carried out by the same manner, with 0 ng, 14 ng, 28 ng and 70 ng of an active form of JNK3 added to the reaction system. Furthermore, in order to test for specificity of JNK3 in the BMAL1 phosphorylation reaction, human JNK1 (143 µU, Upstate Biotechnology), human JNK2 (167 µU, Upstate Biotechnology), or mouse ERK2 (2.7 mU, New England Biolabs) was added to the reaction system instead of JNK3, and then detection of phosphorylated protein was carried out by the same manner. Note that 1 µg of myelin basic protein (hereinafter, MBP) (Sigma) was used as the substrate to confirm the activity of ERK2.

### Results

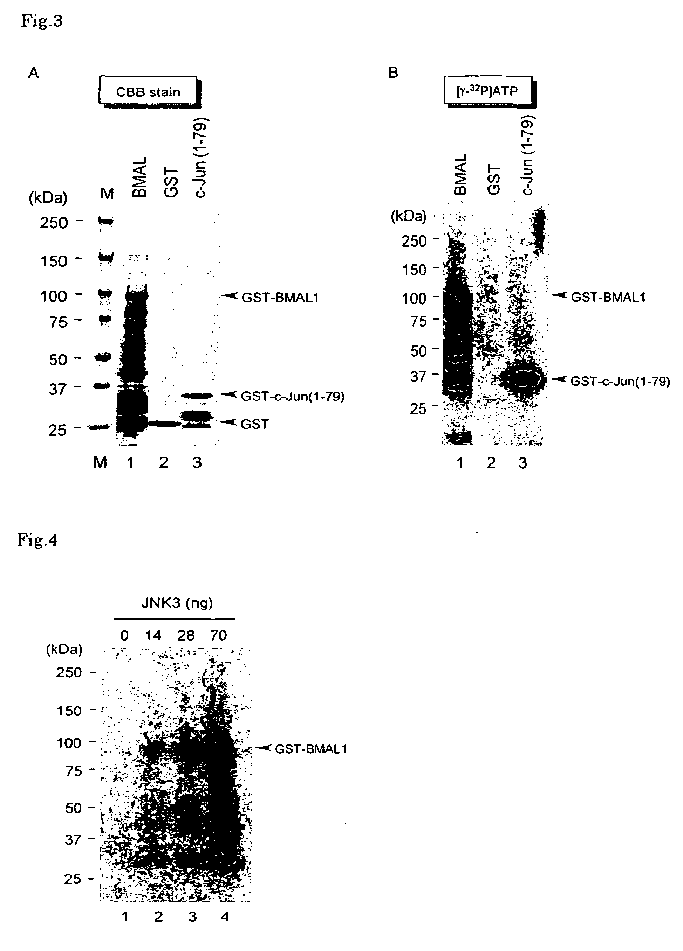

JNK3 did phosphorylate the GST-c-Jun (1-79) used as a positive control but did not phosphorylate the GST (Figure 3B). The experimental system used in the present example was thus confirmed to be suitable for measurement of JNK3 activity. The phosphorylation of GST-BMAL1 was observed in this experimental system (Figure 3B). As shown in Figure 4, GST-BMAL1 was phosphorylated in a JNK3 dose-dependent manner. It was thus understood that phosphorylation of GST-BMAL was not auto-phosphorylation, but was rather phosphorylation by JNK3. Furthermore, GST-BMAL1 was phosphorylated by JNK3, but was substantially not phosphorylated by any of JNK1 and JNK2 (which are members of the JNK family), and ERK2 (which is a member of the MAPK family) (Figure 5). It was thus understood that GST-BMAL1 was phosphorylated in a JNK3 specific manner.

### Experimental example 1

### Suppression of BMAL1/CLOCK dependent transcription by JNK3

BMAL1 is known as a transcription factor that modulates the expression of the Per gene by forming a heterodimer with CLOCK to bind to the E-box (5'-CACGTG-3') present in the 5' upstream region of the Per gene (Gekakis N. *et al., Science,* 1998, Vol. 280, pp.1564-1569). Thus, in order to analyze the interaction between JNK3 and BMAL1 in the cell, a reporter assay was used to test whether the ability of the BMAL1/CLOCK heterodimer for transcriptional activation was modified by the activation of JNK3. Here, it has been known that JNK3 is activated by a cascade wherein MKK4/MKK7 that has been phosphorylated and activated by MEKK1 phosphorylates JNK3. Accordingly, it was analyzed whether the activity (ability for transcriptional activation) of the BMAL1/CLOCK complex was varied by activating JNK3 by way of coexpression of an active form of MEKK 1 or an active form of MKK7.

### Materials

A mammalian expression plasmid for human CLOCK (pCI-CLOCK) was constructed by inserting the ORF region of human CLOCK (which was amplified by PCR using a cDNA clone containing the ORF region of human CLOCK, HG01015 (KIAA0334, provided by Kazusa DNA Research Institute), as a template) into pCI (Promega) which is a mammalian expression vector. It was confirmed that there were no PCR errors in the ORF region, by sequencing using the Big Dye Terminator Cycle Sequencing Kit and the ABI 3100 Genetic Analyzer (both by Applied Biosystems).

A reporter plasmid (pGL3P-M34×3) for detecting the transcriptional activity of the BMAL1/CLOCK complex was constructed by referring to the reports given by Hogenesch *et al., Proceedings of the National Academy of Sciences of the United States of America,* 1998, Vol. 95, p. 5474; *Journal of Neuroscience,* 2000, Vol. 20: RC83, p. 1. Specifically, it was constructed by inserting an M34 × 3 sequence (which contains three E-boxes (5'-GGA CAC GTG ACC ATT GGT CAC GTG TCC ATT GGA CAC GTG ACC-3'; the E-boxes are indicated by underlining) (SEQ ID NO: 4)) into upstream of the promoter of pGL3P (which have an SV40 promoter and a luciferase gene downstream thereof). First, an M34 × 3 DNA fragment having BMAL1/CLOCK recognition sequences in three places was produced in the following manner; synthetic oligo-DNA M34×3-S (5'-GAT CGG ACA CGT GAC CAT TGG TCA CGT GTC CAT TGG ACA CGT GAC C-3') (SEQ ID NO: 5) and M34×3-A (5'-GAT CGG TCA CGT GTC CAA TGG ACA CGT GAC CAA TGG TCA CGT GTC C-3') (SEQ ID NO: 6) were heated at 100°C for 2 minutes and then 70°C for I hour in STE (10 mM Tris-HCl, pH 7.5/1 mM EDTA/100 mM NaCl), subsequently this was gradually returned to room temperature so as to anneal them to form the fragment (dsM34×3). After phosphorylation of the 5' end of the dsM34×3 produced with T4 polynucleotide kinase (New England Biolabs), it was inserted into a pGL3 promoter (Promega) at the Bgl, site (which is a luciferase reporter plasmid) to construct a pGL3P-M34x3. Sequencing was used to confirm that the M34×3 sequence had been inserted with the correct orientation.

pcDNA3.1-BMAL1/V5-His (C-terminal V5/His-tagged, Invitrogen) and pCI-CLOCK (native type, see above) were used for the BMAL1 expression plasmid and the CLOCK expression plasmid. The reporter plasmid, pGL3P-M34x3 (see above) (which is a firefly luciferase reporter plasmid containing three E-boxes that were inserted into upstream of the SV40 promoter) was used for detecting the activity of the BMAL1/CLOCK complex.

pFC-MEKK (Stratagene) was used as an expression plasmid for active form of MEKK1.

SRα-MKK7-DED, which is an expression plasmid for the active form of MKK7, was constructed by inserting a mutant of the MKK7 gene (GenBank; Accession No. AB005654) into an ordinary mammalian expression vector, where the mutant (SEQ ID NO: 1) is that in which the bases of Nos. 859-860, TC, are changed to GA, the bases Nos. 871-873, ACA, are changed to GAG and the bases Nos. 877-879, AGT, are changed to GAC in the region encoding the MKK7 gene.

JBD-JIP1, which is a mammalian expression plasmid for the JNK binding domain of JIP1, was produced according to a method described in the literature (Dickens *et al., Science,* 1997, Vol. 277, pp. 693-696).

JBD-JSAP1, which is a mammalian expression plasmid for the JNK binding domain of JSAP1, was produced using the Echo cloning system (Invitrogen). First, the JBD of the target JSAP-1b gene was inserted into pUni/V5-His-TOPO, then the mammalian expression vector was constructed by recombination using pcDNA 3.1-E as the adapted vector.

A JNK3/293 cell was used for transfection of the plasmid. The cell is a 293EcR cell (293 cell expressing the ecdysone receptor) into which Flag-JNK3/pcDNA 3.1 has been introduced and shows stable expression. However, JNK3 expressed is an inactive form that is not being activated.

### Method

The transfection and the reporter assay were performed as follows. 3 x 10⁵ JNK3/293EcR cells were cultured overnight in 6-well plate, and then used for transfection with FuGENE6 (Roche Diagnostics). pcDNA3.1-BMAL1/V5-His (300 ng), pCI-CLOCK (400 ng), pGL3P-M34x3 (10 ng), pFC-MEKK (0.1-2.0 ng) and SRα-MKK7-DED (20-300 ng) were used as plasmids, and ph RL-CMV (0.1 ng, Promega), which is an expression plasmid for Renilla Luciferase, was used as an internal control. Furthermore, pcDNA3.1 (+) (Invitrogen) was used to adjust the total amount of DNA to 1.0 µg. In order to test the dominant negative effect on JNK, pFC-MEKK (0.5 ng) and (JBD-JIP1 or JBD-JSAP1 (both 50-300 ng)) were used. After culturing for 48 hours, the luciferase activity was measured using the Dual-Luciferase Reporter Assay System (Promega). Note that the measured value was corrected with Renilla luciferase activity. Each experiment was performed three times, in independent duplicates, and the results were expressed with standard error.

### Results

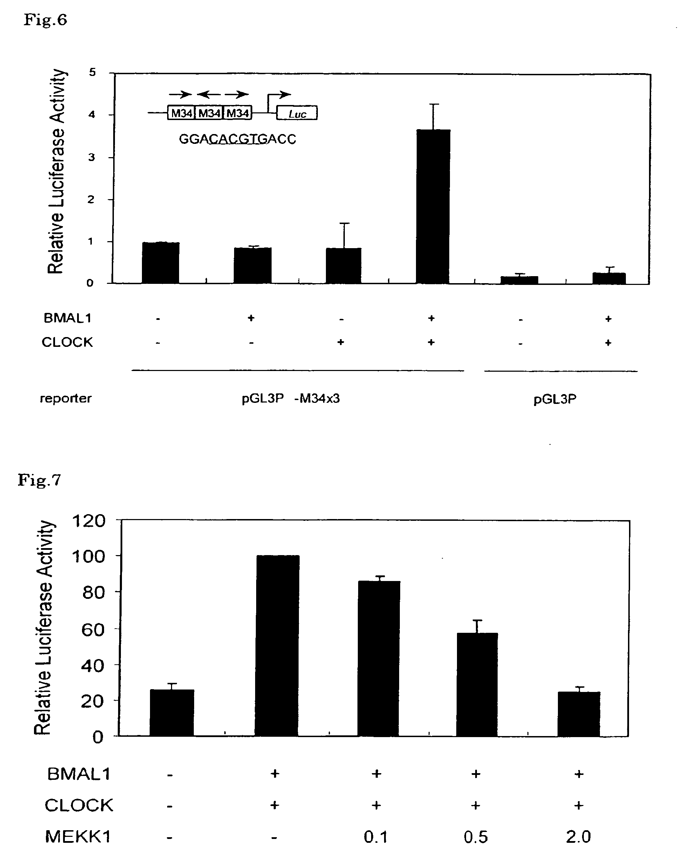

Enhanced transcriptional activity was observed in JNK3/293EcR cells only in the presence of both BMAL1 and CLOCK proteins, from which it was confirmed that the assay system used in the present experimental example was suitable (Figure 6). Furthermore, the enhancement of transcriptional activity was not observed when using a reporter plasmid (pGL3P) that did not contain the BMAL1/CLOCK recognition sequence (Figure 6). In the figure, luciferase activity was shown by relative values to the luciferase activity derived from pGL3P-M34x3 in the absence of BMAL1 and CLOCK taken as a value of 1.

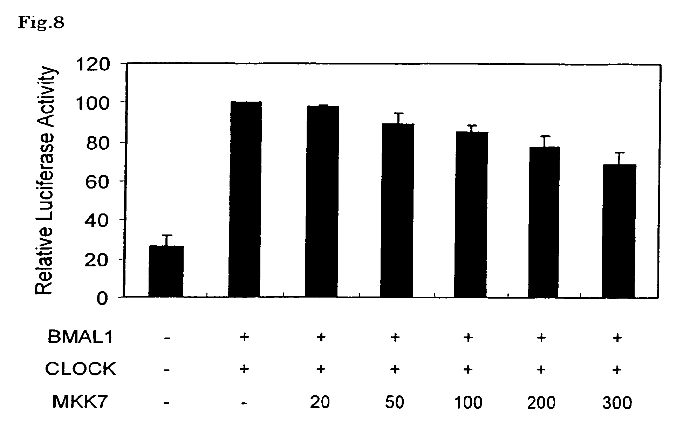

In this experimental system, when the expression plasmid for an active form of MEKK1 or an active form of MKK7 were coexpressed so as to activate JNK3, it was observed that the activity of the BMAL1/CLOCK complex was suppressed in a dose-dependent manner according to the amount of these expression plasmids used (Figure 7 and Figure 8). In the figure, luciferase activity was shown by relative values to the luciferase activity in the presence of BMAL1 and CLOCK, but absence of an active form of MKK1, taken as a value of 100.

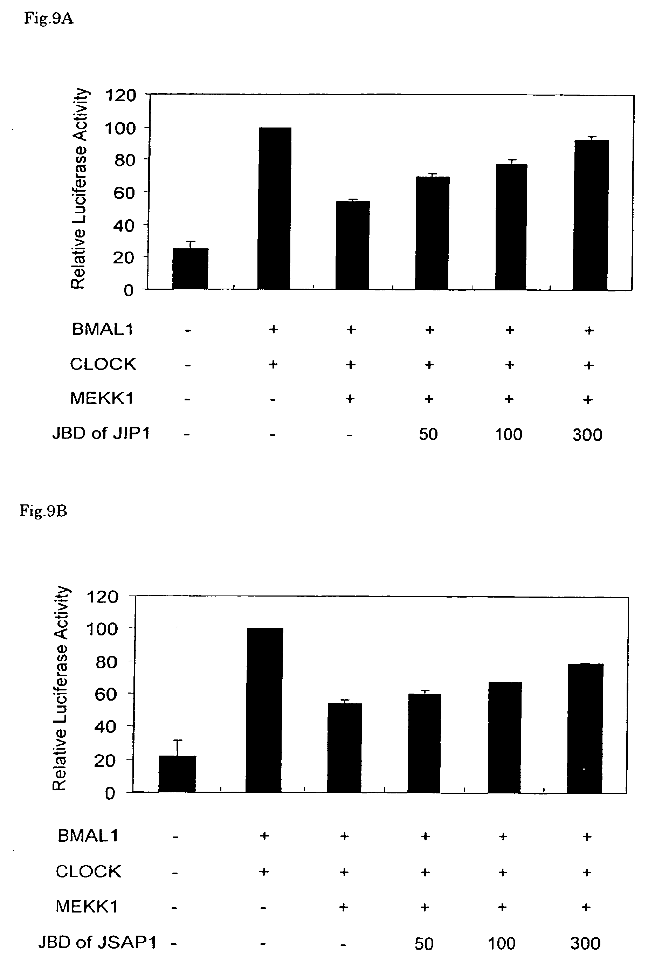

MEKK1 has been reported to be involved in the cascade of ERK and p38. Accordingly, in order to test whether suppression of the activity of the BMAL1/CLOCK complex resulting from coexpression of the active form of MEKK1 was mediated by JNK3, further tests were performed using JIP1 and JSAP1, which are known as scaffold proteins in the JNK cascade. It has been reported that, if only the JNK binding domain (JBD) portions of these proteins are coexpressed with JNK, they show a dominant negative effect on the JNK function (Dickens *et al.*, *Science,* 1997, Vol. 277, pp. 693-696; Ito M. *et al., Molecular and Cellular Biology,* 1999, Vol. 19, p. 7539). The results of the tests on the influence of the coexpression of JBD of JIP1 or JBD of JSAP1 showed that the suppression of activity of the BMAL1/CLOCK complex resulting from the coexpression of an active form of MEKK1 was inhibited by the expression of JBD of JIP1 or JBD of JSAP1 (Figure 9A and Figure 9B). In other words, suppression of the activity of the BMAL1/CLOCK complex resulting from the coexpression of an active form of MEKK1 was found to be mediated by JNK3 that was activated by MEKK1.

These results suggest that BMAL1 is phosphorylated by an active form of JNK3, and as a result, the ability of the BMAL1/CLOCK complex for transcriptional activation is suppressed.

### Possibilities for industrial use

The present invention first discovered the fact that JNK3 interacts with BMAL1, and that BMAL1 is phosphorylated by an active form of JNK3, which results in the suppression of the function thereof. BMAL1 is a transcription factor (that modulates the expression of the Per gene by forming a heterodimer with CLOCK to bind to the E-box (5'-CACGTG-3') present in the 5' upstream region of the Per gene that is a gene implicated in the biological clock) and is involved in a circadian rhythm. Accordingly, if BMAL1 is phosphorylated by JNK3, and the function thereof is suppressed, circadian rhythm disorders arise. In other words, circadian rhythm disorders can be controlled by inhibiting phosphorylation of BMAL1 by JNK3.

Based on these findings, the present invention is able to provide a method for controlling circadian rhythm disorders and a method for treating and/or preventing diseases caused by the disorders, as well as an agent for controlling circadian rhythm disorders and an agent for treating and/or preventing diseases caused by the disorders.

The diseases caused by circadian rhythm disorders are exemplified by sleep/wakefulness rhythm disorders, cyclic/recurrent disorders, and the like. However, it is not limited to these disorders. Examples of sleep/wakefulness rhythm disorders include delayed sleep phase syndrome and non-24-hour sleep patterns. Examples of cyclical/recurrent disorders include endogenous manic depressive psychosis, seasonal affective disorder, cyclic catatonia, cyclic high blood pressure, cyclic ulcers, irregular ovulation cycles, and diabetes caused by cyclic abnormalities in insulin secretion.

Furthermore, circadian rhythm disorders are thought to be associated with nocturnal wandering in cerebrovascular dementia and Alzheimer's dementia. In addition, stress, chronic fatigue, lowered resistance to infection, jet lag, and the like can be ascribed to circadian rhythm disorders. Furthermore, circadian rhythm disorders are sometimes implicated in the efficacy of drugs and the incidence of side effects when administering the drug.

Thus, the present invention is extremely useful in the control of circadian rhythm disorders, the treatment and/or prevention of diseases caused by the disorders and in research into circadian rhythm disorders.

### FREE TEXT in SEQUENCE LISTING

SEQ ID NO 2: Partial peptide of JNK3, which is highly homologous to that (SEQ ID NO:3) of BMAL1
SEQ ID NO 3: Partial peptide of BMAL1, which is highly homologous to that (SEQ ID NO:2) of JNK3
SEQ ID NO 4: Designed oligonucleotide having three E-boxs
SEQ ID NO 5: Designed oligonucleotide for constructing double strand DNA having three E-boxs
SEQ ID NO 6: Designed oligonucleotide for constructing double strand DNA having three E-boxs
SEQ ID NO 7: Partial oligopeptide of JNK3 showing high score in the local alignment between JNK3 and BMAL1
SEQ ID NO 8: Partial oligopeptide of BMAL1 showing high score in the local alignment between JNK3 and BMAL1
SEQ ID NO 9: Partial oligopeptide of JNK3 showing high score in the local alignment between JNK3 and BMAL1
SEQ ID NO 10: Partial oligopeptide of BMAL1 showing high score in the local alignment between JNK3 and BMAL1
SEQ ID NO 11: Partial oligopeptide of JNK3 showing high score in the local alignment between JNK3 and BMAL1
SEQ ID NO 12: Partial oligopeptide of BMAL1 showing high score in the local alignment between JNK3 and BMAL1
SEQ ID NO 13: Partial oligopeptide of JNK3 showing high score in the local alignment between JNK3 and BMAL1
SEQ ID NO 14: Partial oligopeptide of BMAL1 showing high score in the local alignment between JNK3 and BMAL1
SEQ ID NO 15: Partial oligopeptide of JNK3 showing high score in the local alignment between JNK3 and BMAL1
SEQ ID NO 16: Partial oligonucleotide of BMAL1 showing a high score in local the alignment of JNK3 and BMAL1
SEQ ID NO 17: Partial oligopeptide of JNK3 showing a high score in local the alignment of JNK3 and BMAL1
SEQ ID NO 18: Partial oligonucleotide of BMAL1 showing a high score in local the alignment of JNK3 and BMAL1
SEQ ID NO 19: Partial oligopeptide of JNK3 showing a high score in local the alignment of JNK3 and BMAL1
SEQ ID NO 20: Partial oligonucleotide of BMAL1 showing a high score in local the alignment of JNK3 and BMAL1
SEQ ID NO 21: Partial oligopeptide of JNK3 showing a high score in local the alignment of JNK3 and BMAL2
SEQ ID NO 22: Partial oligonucleotide of BMAL2 showing a high score in local the alignment of JNK3 and BMAL2
SEQ ID NO 23: Partial oligopeptide of JNK3 showing a high score in local the alignment of JNK3 and BMAL2
SEQ ID NO 24: Partial oligonucleotide of BMAL2 showing a high score in local the alignment of JNK3 and BMAL2
SEQ ID NO 25: Partial oligopeptide of JNK3 showing a high score in local the alignment of JNK3 and BMAL2
SEQ ID NO 26: Partial oligonucleotide of BMAL2 showing a high score in local the alignment of JNK3 and BMAL2
SEQ ID NO 27: Partial oligopeptide of JNK3 showing a high score in local the alignment of JNK3 and BMAL2
SEQ ID NO 28: Partial oligonucleotide of BMAL2 showing a high score in local the alignment of JNK3 and BMAL2

## Claims

1. Use of an inhibitor of the phosphorylation of BMAL1 by c-Jun N-terminal kinase 3 (JNK3) for the manufacture of an agent for controlling a circadian rhythm disorder, wherein said inhibitor is selected from the group of antibodies that inhibit the interaction between JNK3 and BMAL1, antisense oligonucleotides of the JNK3 gene, and the JBD of JIP1 or JSAP1.

2. Use according to claim 1, wherein the agent is a medicament for controlling a circadian rhythm disorder.

3. Use of an inhibitor of the phosphorylation of BMAL1 by c-Jun N-terminal kinase 3 (JNK3) for the manufacture of a medicament for treating and/or preventing a disease caused by a circadian rhythm disorder, wherein said inhibitor is selected from the group of antibodies that inhibit the interaction between JNK3 and BMAL1, antisense oligonucleotides of the JNK3 gene, and the JBD of JIP1 or JSAP1.

4. Use according to any of claims 1 to 3, wherein the circadian rhythm disorder is a sleep/wakefulness rhythm disorder and/or a cyclical/recurrent disorder.

5. A method for identifying a compound that inhibits the interaction between c-Jun N-terminal kinase 3 (JNK3) and BMAL1, wherein the method comprises contacting a compound with JNK3 and/or BMAL1 under conditions allowing for the interaction of the compound with JNK3 and/or BMAL1, and determining whether the compound inhibits the interaction between JNK3 and BMAL1 by using a system that uses a signal and/or a marker generated by the interaction between JNK3 and BMAL1 to detect presence or absence or change of the signal and/or the marker.

6. A method for identifying a compound that inhibits the phosphorylation of BMAL1 by c-Jun N-terminal kinase 3 (JNK3), wherein the method comprises contacting a compound with JNK3 and/or BMAL1, and determining whether the compound inhibits the phosphorylation of BMAL1 by JNK3, by using a system that uses a signal and/or a marker capable of detecting the phosphorylation of BMAL1 to detect presence or absence or change of this signal and/or marker.

7. An in-vitro method for recovering the suppressed transcriptional activity of a complex comprising BMAL1 and CLOCK, wherein the method comprises inhibiting the expression of c-Jun N-terminal kinase 3 and/or inhibiting the function of c-Jun N-terminal kinase 3.

8. A reagent kit, wherein said kit comprises at least one member selected from the group consisting of BMAL1, a polynucleotide encoding BMAL1 and a vector comprising a polynucleotide encoding BMAL; at least one member selected from the group consisting of c-Jun N-terminal kinase 3 (JNK3), a polynucleotide encoding JNK3 and a vector comprising a polynucleotide encoding JNK3.

## Patentansprüche

1. Verwendung eines Inhibitors der Phosphorylierung von BMAL1 durch die c-Jun N-terminale Kinase 3 (JNK3) zur Herstellung eines Mittels zur Kontrolle einer Störung des Tagesrhythmus, wobei der Inhibitor ausgewählt ist aus der Gruppe von Antikörpern, die die Interaction zwischen JNK3 und BMAL1 inhibieren, antisense Oligonukleotiden des Gens für JNK3, und der JBD von JIP1 oder JSAP1.

2. Verwendung nach Anspruch 1, wobei das Mittel ein Medikament zur Kontrolle einer Störung des Tagesrhythmus ist.

3. Verwendung eines Inhibitors der Phosphorylierung von BMAL1 durch die c-Jun N-terminale Kinase 3 (JNK3) zur Herstellung eines Medikaments zur Behandlung und/oder Vorbeugung einer Erkrankung, die durch eine Störung des Tagesrhythmus verursacht wird, wobei der Inhibitor ausgewählt ist aus der Gruppe von Antikörpern, die die Interaction zwischen JNK3 und BMAL1 inhibieren, antisense Oligonukleotiden des Gens für JNK3, und der JBD von JIP1 oder JSAP1.

4. Verwendung nach einem der Ansprüche 1 bis 3, wobei die Störung des Tagesrhythmus eine Störung des Schlaf/Wachrhythmus und/oder eine zyklische/sich wiederholende Störung ist.

5. Verfahren zur Identifizierung einer Verbindung, die die Interaktion zwischen c-Jun N-terminaler Kinase 3 (JNK3) und BMAL1 inhibiert, wobei das Verfahren ein in Kontakt bringen einer Verbindung mit JNK3 und/oder BMAL1 unter Bedingungen, die die Interaktion der Verbindung mit JNK3 und/oder BMAL1 ermöglichen, und Bestimmen, ob die Verbindung die Interaktion zwischen JNK3 and BMAL1 inhibiert, unter Verwendung eines Systems, das ein Signal und/oder einen Marker verwendet, der durch die Interaktion zwischen JNK3 and BMAL1 erzeugt wird, um die Anwesenheit oder Abwesenheit oder Veränderung des Signals und/oder des Markers nachzuweisen, umfaßt.

6. Verfahren zur Identifizierung einer Verbindung, die die Phosphorylierung von BMAL1 durch c-Jun N-terminale Kinase 3 (JNK3) inhibiert, wobei das Verfahren ein in Kontakt bringen einer Verbindung mit JNK3 und/oder BMAL1, und Bestimmen ob die Verbindung die Phosphorylierung von BMAL1 durch JNK3 inhibiert, unter Verwendung eines Systems, das ein Signal und/oder einen Marker verwendet, das/der in der Lage ist, die Phosphorylierung of BMAL1 nachzuweisen, um die Anwesenheit oder Abwesenheit oder Veränderung dieses Signals und/oder des Markers nachzuweisen, umfaßt.

7. In-vitro Verfahren zum Rückerhalten der unterdrückten transkriptionellen Aktivität eines Komplexes umfassend BMAL1 und CLOCK, wobei das Verfahren ein Inhibieren der Expression von c-Jun N-terminaler Kinase 3 und/oder ein Inhibieren der Funktion von c-Jun N-terminaler Kinase 3 umfaßt.

8. Reagenzkit, wobei der Kit mindestens ein Mitglied ausgewählt aus der Gruppe bestehend aus BMAL1, einem Polynukleotid das für BMAL1 kodiert und einem Vektor umfassend ein Polynukleotid das für BMAL kodiert; mindestens ein Mitglied ausgewählt aus der Gruppe bestehend aus c-Jun N-terminaler Kinase 3 (JNK3), einem Polynukleotid das für JNK3 kodiert und einem Vektor umfassend ein Polynukleotid das für JNK3 kodiert, umfaßt.

## Revendications

1. Utilisation d'un inhibiteur de la phosphorylation de BMAL1 par la kinase 3 N-terminale de c-Jun (JNK3) pour la fabrication d'un agent pour le contrôle d'un trouble du rythme circadien, dans laquelle ledit inhibiteur est choisi dans le groupe des anticorps qui inhibent l'interaction entre JNK3 et BMAL1, des oligonucléotides antisens du gène de JNK3 et de JBD de JIP1 ou JSAP1.

2. Utilisation selon la revendication 1, dans laquelle l'agent est un médicament destiné à contrôler un trouble du rythme circadien.

3. Utilisation d'un inhibiteur de la phosphorylation de BMAL1 par la kinase 3 N-terminale de c-Jun (JNK3) pour la fabrication d'un médicament pour le traitement et/ou la prévention d'une maladie provoquée par un trouble du rythme circadien, dans laquelle ledit inhibiteur est choisi dans le groupe des anticorps qui inhibent l'interaction entre JNK3 et BMAL1, des oligonucléotides antisens du gène de JNK3 et de JBD de JIP1 ou JSAP1.

4. Utilisation selon l'une quelconque des revendications 1 à 3, dans laquelle le trouble du rythme circadien est un trouble du rythme sommeil/veille et/ou un trouble cyclique/récurrent.

5. Procédé d'identification d'un composé qui inhibe l'interaction entre la kinase 3 N-terminale de c-Jun (JNK3) et BMAL1, le procédé comprenant la mise en contact d'un composé avec JNK3 et/ou BMAL1 dans des conditions permettant l'interaction du composé avec JNK3 et/ou BMAL1, et la détermination du fait que le composé inhibe ou non l'interaction entre JNK3 et BMAL1 en utilisant un système qui utilise un signal et/ou un marqueur généré par l'interaction entre JNK3 et BMAL1 pour détecter la présence ou l'absence ou le changement du signal et/ou du marqueur.

6. Procédé d'identification d'un composé qui inhibe la phosphorylation de BMAL1 par la kinase 3 N-terminale de c-Jun (JNK3), le procédé comprenant la mise en contact d'un composé avec JNK3 et/ou BMAL1 et la détermination du fait que le composé inhibe ou non la phosphorylation de BMAL1 par JNK3, en utilisant un système qui utilise un signal et/ou un marqueur capable de détecter la phosphorylation de BMAL1, afin de détecter la présence ou l'absence ou le changement de ce signal et/ou marqueur.

7. Procédé in vitro de récupération de l'activité transcriptionnelle supprimée d'un complexe comprenant BMAL1 et CLOCK, le procédé comprenant l'inhibition de l'expression de la kinase 3 N-terminale de c-Jun et/ou l'inhibition de la fonction de la kinase 3 N-terminale de C-Jun.

8. Trousse de réactif, ladite trousse comprenant au moins un élément choisi dans le groupe constitué par BMAL1, un polynucléotide codant BMAL1 et un vecteur comprenant un polynucléotide codant BMAL1 ; au moins un élément choisi dans le groupe constitué par la kinase 3 N-terminale de c-Jun (JNK3), un polynucléotide codant JNK3 et un vecteur comprenant un polynucléotide codant JNK3.


### SEQUENCE LISTING

<110> CELESTAR LEXICO-SCIENCES, INC. DAIICHI PHARMACEUTICAL CO., LTD.
<120> Agent for controlling circadian rhythm failure
<130> FB14182
<150> JP P2002-022857
   <151> 2002-01-31
<160> 28
<170> PatentIn version 3.1
<210> 1
   <211> 1407
   <212> DNA
   <213> Mus musculus
<400> 1
   atggcggcgt cctccctgga gcagaagctg tcccgcctgg aagccaagct gaagcaggag 60
   aaccgtgagg cccgcaggag gatcgacctc aacttggata tcagcccaca gcggcccagg 120
   cccattattg tgatcactct aagccctgct cctgccccgt cccagcgagc agccctgcaa 180
   ctcccactgg ccaacgatgg gggcagccgc tcaccatcct cagagagctc cccacagcac 240
   cctacacccc ccacccggcc ccgccacatg ctggggctcc catcaacctt gttcacaccg 300
   cgcagtatgg agagcatcga gattgaccag aagctgcagg agatcatgaa gcagacaggg 360
   tacctgacta tcgggggcca gcgttatcag gcagaaatca atgacttgga gaacttgggt 420
   gagatgggca gtggtacctg tggtcaggtg tggaagatgc ggttccggaa gacaggccac 480
   atcattgctg ttaagcaaat gcggcgctct gggaacaagg aagagaataa gcgcattttg 540
   atggacctgg atgtagtact caagagccat gactgccctt acatcgttca gtgctttggc 600
   accttcatca ccaacacaga cgtctttatt gccatggagc tcatgggcac atgtgcagag 660
   aagctgaaga aacgaatgca gggccccatt ccagagcgaa tcctgggcaa gatgactgtg 720
   gcgattgtga aagcactgta ctatctgaag gagaagcatg gcgtcatcca tcgcgatgtc 780
   aaaccctcca acatcctgct agatgagcgg ggccagatca agctctgtga ctttggcatc 840
   agtggccgcc ttgttgacga caaagccaaa gagcgggacg ctggctgtgc tgcctatatg 900
   gctcccgagc gcatcgaccc tccagatccc accaagcctg actatgacat ccgagctgat 960
   gtgtggagcc tgggcatctc actggtggag ctggcaacag gacagttccc ctataagaac 1020
   tgcaagacgg actttgaggt cctcaccaaa gtcctacagg aagagccccc actcctgcct 1080
   ggtcacatgg gcttctcagg ggacttccag tcatttgtca aagactgcct tactaaagat 1140
   cacaggaaga gaccaaagta taataagcta cttgaacaca gcttcatcaa gcactatgag 1200
   atactcgagg tggatgtcgc gtcctggttt aaggatgtca tggcgaagac cgagtcccca 1260
   aggactagtg gagtcctgag tcagcaccat ctgcccttct tcagtgggag tctggaggag 1320
   tctcccactt ccccaccttc tcccaagtcc ttccctctgt caccagccat ccctcaggcc 1380
   caggcagagt gggtctcggg caggtag 1407
<210> 2
   <211> 6
   <212> PRT
   <213> homo sapiens
<220>
   <221> misc_feature
   <223> Partial peptide of JNK3, which is highly homologous to that (SEQ ID NO:3) of BMAL1 or BMAL2
<400> 2
   Lys Val Ile Glu Gln Leu
   1               5
<210> 3
   <211> 6
   <212> PRT
   <213> homo sapiens
<220>
   <221> misc_feature
   <223> Partial peptide of BMAL1 or BMAL2, which is highly homologous to that (SEQ ID NO:2) of JNK3
<900> 3
<210> 4
   <211> 42
   <212> DNA
   <213> Artificial
<220>
   <223> Designed oligonucleotide having 3 E-boxs
<400> 4
   ggacacgtga ccattggtca cgtgtccatt ggacacgtga cc 42
<210> 5
   <211> 46
   <212> DNA
   <213> Artificial
<220>
   <223> Designed oligonucleotide for constructing double strand DNA havin g 3 E-boxs
<400> 5
   gatcggacac gtgaccattg gtcacgtgtc cattggacac gtgacc 46
<210> 6
   <211> 46
   <212> DNA
   <213> Artificial
<220>
   <223> Designed oligonucleotide for constructing double strand DNA having 3 E-boxs
<400> 6
   gatcggtcac gtgtccaatg gacacgtgac caatggtcac gtgtcc 46
<210> 7
   <211> 23
   <212> PRT
   <213> homo sapiens
<220>
   <221> misc_feature
   <223> Partial oligopeptide of JNK3 showing high score in the local alig nment between JNK3 and BMAL1
<400> 7
   Ser Lys Ser Lys Val Asp Asn Gln Phe Tyr Ser Val Glu Val Gly Asp
   1               5                   10                  15
   Ser Thr Phe Thr Val Leu Lys
               20
<210> 8
   <211> 23
   <212> PRT
   <213> homo sapiens
<220>
   <221> misc_feature
   <223> Partial oligopeptide of BMAL1 showing high score in the local ali gnment between JNK3 and BMAL1
<400> 8
   Thr Arg Glu Lys Ile Thr Thr Asn Cys Tyr Lys Phe Lys Ile Lys Asp
   1               5                   10                  15
   Gly Ser Phe Ile Thr Leu Arg
               20
<210> 9
   <211> 14
   <212> PRT
   <213> homo sapiens
<220>
   <221> misc_feature
   <223> Partial oligopeptide of JNK3 showing high score in the local alig nment between JNK3 and BMAL1
<400> 9
   Val Gly Asp Ser Thr Phe Thr Val Leu Lys Arg Tyr Gln Asn
   1               5                   10
<210> 10
   <211> 14
   <212> PRT
   <213> homo sapiens
<220>
   <221> misc_feature
   <223> Partial oligopeptide of BMAL1 showing high score in the local ali gnment between JNK3 and BMAL1
<400> 10
   Val Ser Glu Ser Val Phe Lys Ile Leu Asn Tyr Ser Gln Asn
   1               5                   10
<210> 11
   <211> 10
   <212> PRT
   <213> homo sapiens
<220>
   <221> misc_feature
   <223> Partial oligopeptide of JNK3 showing high score in the local alig nment between JNK3 and BMAL1
<400> 11
   Glu Gln Leu Gly Thr Pro Cys Pro Glu Phe
   1               5                   10
<210> 12
   <211> 10
   <212> PRT
   <213> homo sapiens
<220>
   <221> misc_feature
   <223> Partial oligopeptide of BMAL1 showing high score in the local ali gnment between JNK3 and BMAL1
<400> 12
   Glu Leu Leu Gly Thr Ser Cys Tyr Glu Tyr
   1               5                   10
<210> 13
   <211> 22
   <212> PRT
   <213> homo sapiens
<220>
   <221> misc_feature
   <223> Partial oligopeptide of JNK3 showing high score in the local alig nment between JNK3 and BMAL1
<400> 13
   Ser Ser Met Ser Thr Asp Gln Thr Leu Ala Ser Asp Thr Asp Ser Ser
   1 5 10 15
   Leu Glu Ala Ser Ala Gly
   20
<210> 14
   <211> 22
   <212> PRT
   <213> homo sapiens
<220>
   <221> misc_feature
   <223> Partial oligopeptide of BMAL1 showing high score in the local ali gnment between JNK3 and BMAL1
<400> 14
   Ser Ser Pro Ser Asn Asp Glu Ala Ala Met Ala Val Ile Met Ser Leu
   1 5 10 15
   Leu Glu Ala Asp Ala Gly
   20
<210> 15
   <211> 10
   <212> PRT
   <213> homo sapiens
<220>
   <221> misc_feature
   <223> Partial oligopeptide of JNK3 showing high score in the local alig nment between JNK3 and BMAL1
<400> 15
   Ser Asp Cys Thr Leu Lys Ile Leu Asp Phe
   1 5 10
<210> 16
   <211> 10
   <212> PRT
   <213> homo sapiens
<220>
   <221> misc_feature
   <223> Partial oligopeptide of BMAL1 showing high score in the local ali gnment between JNK3 and BMAL1
<400> 16
   Ser Glu Ser Val Phe Lys Ile Leu Asn Tyr
   1 5 10
<210> 17
   <211> 10
   <212> PRT
   <213> homo sapiens
<220>
   <221> misc_feature
   <223> Partial oligopeptide of JNK3 showing high score in the local alig nment between JNK3 and BMAL1
<400> 17
   Tyr Ile Asp Gln Trp Asn Lys Val Ile Glu
   1 5 10
<210> 18
   <211> 10
   <212> PRT
   <213> homo sapiens
<220>
   <221> misc_feature
   <223> Partial oligopeptide of BMAL1 showing high score in the local ali gnment between JNK3 and BMAL1
<400> 18
   Phe Met Asn Pro Trp Thr Lys Glu Val Glu
   1 5 10
<210> 19
   <211> 11
   <212> PRT
   <213> homo sapiens
<220>
   <221> misc_feature
   <223> Partial oligopeptide of JNK3 showing high score in the local alig nment between JNK3 and BMAL1
<400> 19
   Val Lys Gly Gln Pro Ser Pro Ser Gly Ala Ala
   1 5 10
<210> 20
   <211> 11
   <212> PRT
   <213> homo sapiens
<220>
   <221> misc_feature
   <223> Partial oligopeptide of BMAL1 showing high score in the local ali gnment between JNK3 and BMAL1
<400> 20
   Val Lys Glu Gln Leu Ser Ser Ser Asp Thr Ala
   1               5                   10
<210> 21
   <211> 31
   <212> PRT
   <213> homo sapiens
<220>
   <221> misc_feature
   <223> Partial oligopeptide of JNK3 showing high score in the local alig nment between JNK3 and BMAL2
<400> 21
   Glu Glu Lys Thr Lys Asn Gly Val Val Lys Gly Gln Pro Ser Pro Ser
   1               5                   10                  15
   Gly Ala Ala Val Asn Ser Ser Glu Ser Leu Pro Pro Ser Ser Ser
               20                  25                  30
<210> 22
   <211> 31
   <212> PRT
   <213> homo sapiens
<220>
   <221> misc_feature
   <223> Partial oligopeptide of BMAL2 showing high score in the local ali gnment between JNK3 and BMAL2
<400> 22
   Asp Asp Ser Ser Pro Thr Gly Leu Met Lys Asp Thr His Thr Val Asn
   1               5                   10                  15
   Cys Arg Ser Met Ser Asn Lys Glu Leu Phe Pro Pro Ser Pro Ser
               20                  25                  30
<210> 23
   <211> 10
   <212> PRT
   <213> homo sapiens
<220>
   <221> misc_feature
   <223> Partial oligopeptide of JNK3 showing high score in the local alig nment between JNK3 and BMAL2
<400> 23
   Glu Gln Leu Gly Thr Pro Cys Pro Glu Phe
   1 5 10
<210> 24
   <211> 10
   <212> PRT
   <213> homo sapiens
<220>
   <221> misc_feature
   <223> Partial oligopeptide of BMAL2 showing high score in the local ali gnment between JNK3 and BMAL2
<400> 24
   Glu Leu Leu Gly Thr Ser Cys Tyr Glu Tyr
   1 5 10
<210> 25
   <211> 23
   <212> PRT
   <213> homo sapiens
<220>
   <221> misc_feature
   <223> Partial oligopeptide of JNK3 showing high score in the local alig nment between JNK3 and BMAL2
<400> 25
   Ser Lys Ser Lys Val Asp Asn Gln Phe Tyr Ser Val Glu Val Gly Asp
   1 5 10 15
   Ser Thr Phe Thr Val Leu Lys
   20
<210> 26
   <211> 23
   <212> PRT
   <213> homo sapiens
<220>
   <221> misc_feature
   <223> Partial oligopeptide of BMAL2 showing high score in the local ali gnment between JNK3 and BMAL2
<400> 26
   Ser Lys Glu Lys Ile Leu Thr Asp Ser Tyr Lys Phe Arg Ala Lys Asp
   1 5 10 15
   Gly Ser Phe Val Thr Leu Lys
   20
<210> 27
   <211> 12
   <212> PRT
   <213> homo sapiens
<220>
   <221> misc_feature
   <223> Partial oligopeptide of JNK3 showing high score in the local alig nment between JNK3 and BMAL2
<400> 27
   Ser Lys Ser Lys Val Asp Asn Gln Phe Tyr Ser Val
   1 5 10
<210> 28
   <211> 12
   <212> PRT
   <213> homo sapiens
<220>
   <221> misc_feature
   <223> Partial oligopeptide of BMAL2 showing high score in the local ali gnment between JNK3 and BMAL2
<400> 28
   Ser Lys Lys Lys Glu His Arg Lys Phe Tyr Thr Ile
   1 5 10

### SEQUENCE LISTING

<110> CELESTAR LEXICO-SCIENCES, INC. DAIICHI PHARMACEUTICAL CO., LTD.
<120> Agent for controlling circadian rhythm failure
<130> FB14182
<150> JP P2002-022857
   <151> 2002-01-31
<160> 28
<170> PatentIn version 3.1
<210> 1
   <211> 1407
   <212> DNA
   <213> Mus musculus
<400> 1

### SEQUENCE LISTING

<110> CELESTAR LEXICO-SCIENCES, INC. DAIICHI PHARMACEUTICAL CO., LTD.
<120> Agent for controlling a circadian rhythm failure
<130> FB14182
<150> JP P2002-022857
   <151> 2002-01-31
<160> 28
<170> PatentIn version 3.1
<210> 1
   <211> 1407
   <212> DNA
   <213> Mus musculus
<400> 1
   atggcggcgt cctccctgga gcagaagctg tcccgcctgg aagccaagct gaagcaggag      60
   aaccgtgagg cccgcaggag gatcgacctc aacttggata tcagcccaca gcggcccagg     120
   cccattattg tgatcactct aagccctgct cctgccccgt cccagcgagc agccctgcaa     180
   ctcccactgg ccaacgatgg gggcagccgc tcaccatcct cagagagctc cccacagcac     240
   cctacacccc ccacccggcc ccgccacatg ctggggctcc catcaacctt gttcacaccg     300
   cgcagtatgg agagcatcga gattgaccag aagctgcagg agatcatgaa gcagacaggg     360
   tacctgacta tcgggggcca gcgttatcag gcagaaatca atgacttgga gaacttgggt     420
   gagatgggca gtggtacctg tggtcaggtg tggaagatgc ggttccggaa gacaggccac     480
   atcattgctg ttaagcaaat gcggcgctct gggaacaagg aagagaataa gcgcattttg     540
   atggacctgg atgtagtact caagagccat gactgccctt acatcgttca gtgctttggc     600
   accttcatca ccaacacaga cgtctttatt gccatggagc tcatgggcac atgtgcagag     660
   aagctgaaga aacgaatgca gggccccatt ccagagcgaa tcctgggcaa gatgactgtg     720
   gcgattgtga aagcactgta ctatctgaag gagaagcatg gcgtcatcca tcgcgatgtc     780
   aaaccctcca acatcctgct agatgagcgg ggccagatca agctctgtga ctttggcatc     840
   agtggccgcc ttgttgacga caaagccaaa gagcgggacg ctggctgtgc tgcctatatg     900
   gctcccgagc gcatcgaccc tccagatccc accaagcctg actatgacat ccgagctgat     960
   gtgtggagcc tgggcatctc actggtggag ctggcaacag gacagttccc ctataagaac    1020
   tgcaagacgg actttgaggt cctcaccaaa gtcctacagg aagagccccc actcctgcct    1080
   ggtcacatgg gcttctcagg ggacttccag tcatttgtca aagactgcct tactaaagat    1140
   cacaggaaga gaccaaagta taataagcta cttgaacaca gcttcatcaa gcactatgag    1200
   atactcgagg tggatgtcgc gtcctggttt aaggatgtca tggcgaagac cgagtcccca    1260
   aggactagtg gagtcctgag tcagcaccat ctgcccttct tcagtgggag tctggaggag 1320
   tctcccactt ccccaccttc tcccaagtcc ttccctctgt caccagccat ccctcaggcc 1380
   caggcagagt gggtctcggg caggtag 1407
<210> 2
   <211> 6
   <212> PRT
   <213> homo sapiens
<220>
   <221> misc_feature
   <223> Partial peptide of JNK3, which is highly homologous to that (SEQ ID NO:3) of BMAL1 or BMAL2
<400> 2
   Lys Val Ile Glu Gln Leu
   1 5
<210> 3
   <211> 6
   <212> PRT
   <213> homo sapiens
<220>
   <221> misc_feature
   <223> Partial peptide of BMAL1 or BMAL2, which is highly homologous to that (SEQ ID NO:2) of JNK3
<400> 3
   Lys Val Lys Glu Gln Leu
   1 5
<210> 4
   <211> 42
   <212> DNA
   <213> Artificial
<220>
   <223> Designed oligonucleotide having 3 E-boxs
<400> 4
   ggacacgtga ccattggtca cgtgtccatt ggacacgtga cc 42
<210> 5
   <211> 46
   <212> DNA
   <213> Artificial
<220>
   <223> Designed oligonucleotide for constructing double strand DNA havin g 3 E-boxs
<400> 5
   gatcggacac gtgaccattg gtcacgtgtc cattggacac gtgacc 46
<210> 6
   <211> 46
   <212> DNA
   <213> Artificial
<220>
   <223> Designed oligonucleotide for constructing double strand DNA havin g 3 E-boxs
<400> 6
   gatcggtcac gtgtccaatg gacacgtgac caatggtcac gtgtcc 46
<210> 7
   <211> 23
   <212> PRT
   <213> homo sapiens
<220>
   <221> misc_feature
   <223> Partial oligopeptide of JNK3 showing high score in the local alig nment between JNK3 and BMAL1
<400> 7
   Ser Lys Ser Lys Val Asp Asn Gln Phe Tyr Ser Val Glu Val Gly Asp
   1               5                   10                  15
   Ser Thr Phe Thr Val Leu Lys
               20
<210> 8
   <211> 23
   <212> PRT
   <213> homo sapiens
<220>
   <221> misc_feature
   <223> Partial oligopeptide of BMAL1 showing high score in the local ali gnment between JNK3 and BMAL1
<400> 8
   Thr Arg Glu Lys Ile Thr Thr Asn Cys Tyr Lys Phe Lys Ile Lys Asp
   1               5                   10                  15
   Gly Ser Phe Ile Thr Leu Arg
               20
<210> 9
   <211> 14
   <212> PRT
   <213> homo sapiens
<220>
   <221> misc_feature
   <223> Partial oligopeptide of JNK3 showing high score in the local alig nment between JNK3 and BMAL1
<400> 9
   Val Gly Asp Ser Thr Phe Thr Val Leu Lys Arg Tyr Gln Asn
   1               5                   10
<210> 10
   <211> 14
   <212> PRT
   <213> homo sapiens
<220>
   <221> misc_feature
   <223> Partial oligopeptide of BMAL1 showing high score in the local ali gnment between JNK3 and BMAL1
<400> 10
   Val Ser Glu Ser Val Phe Lys Ile Leu Asn Tyr Ser Gln Asn
   1               5                   10
<210> 11
   <211> 10
   <212> PRT
   <213> homo sapiens
<220>
   <221> misc_feature
   <223> Partial oligopeptide of JNK3 showing high score in the local alig nment between JNK3 and BMAL1
<400> 11
   Glu Gln Leu Gly Thr Pro Cys Pro Glu Phe
   1               5                   10
<210> 12
   <211> 10
   <212> PRT
   <213> homo sapiens
<220>
   <221> misc_feature
   <223> Partial oligopeptide of BMAL1 showing high score in the local ali gnment between JNK3 and BMAL1
<400> 12
   Glu Leu Leu Gly Thr Ser Cys Tyr Glu Tyr
   1               5                   10
<210> 13
   <211> 22
   <212> PRT
   <213> homo sapiens
<220>
   <221> misc_feature
   <223> Partial oligopeptide of JNK3 showing high score in the local alig nment between JNK3 and BMAL1
<400> 13
   Ser Ser Met Ser Thr Asp Gln Thr Leu Ala Ser Asp Thr Asp Ser Ser
   1               5                   10                  15
   Leu Glu Ala Ser Ala Gly
               20
<210> 14
   <211> 22
   <212> PRT
   <213> homo sapiens
<220>
   <221> misc_feature
   <223> Partial oligopeptide of BMAL1 showing high score in the local ali gnment between JNK3 and BMAL1
<400> 14
   Ser Ser Pro Ser Asn Asp Glu Ala Ala Met Ala Val Ile Met Ser Leu
   1               5                   10                  15
   Leu Glu Ala Asp Ala Gly
               20
<210> 15
   <211> 10
   <212> PRT
   <213> homo sapiens
<220>
   <221> misc_feature
   <223> Partial oligopeptide of JNK3 showing high score in the local alig nment between JNK3 and BMAL1
<400> 15
   Ser Asp Cys Thr Leu Lys Ile Leu Asp Phe
   1               5                   10
<210> 16
   <211> 10
   <212> PRT
   <213> homo sapiens
<220>
   <221> misc_feature
   <223> Partial oligopeptide of BMAL1 showing high score in the local ali gnment between JNK3 and BMAL1
<400> 16
   Ser Glu Ser Val Phe Lys Ile Leu Asn Tyr
   1               5                   10
<210> 17
   <211> 10
   <212> PRT
   <213> homo sapiens
<220>
   <221> misc_feature
   <223> Partial oligopeptide of JNK3 showing high score in the local alig nment between JNK3 and BMAL1
<400> 17
   Tyr Ile Asp Gln Trp Asn Lys Val Ile Glu
   1               5                   10
<210> 18
   <211> 10
   <212> PRT
   <213> homo sapiens
<220>
   <221> misc_feature
   <223> Partial oligopeptide of BMAL1 showing high score in the local ali gnment between JNK3 and BMAL1
<400> 18
   Phe Met Asn Pro Trp Thr Lys Glu Val Glu
   1               5                   10
<210> 19
   <211> 11
   <212> PRT
   <213> homo sapiens
<220>
   <221> misc_feature
   <223> Partial oligopeptide of JNK3 showing high score in the local alig nment between JNK3 and BMAL1
<400> 19
   Val Lys Gly Gln Pro Ser Pro Ser Gly Ala Ala
   1               5                   10
<210> 20
   <211> 11
   <212> PRT
   <213> homo sapiens
<220>
   <221> misc_feature
   <223> Partial oligopeptide of BMAL1 showing high score in the local ali gnment between JNK3 and BMAL1
<400> 20
   Val Lys Glu Gln Leu Ser Ser Ser Asp Thr Ala
   1               5                   10
<210> 21
   <211> 31
   <212> PRT
   <213> homo sapiens
<220>
   <221> misc_feature
   <223> Partial oligopeptide of JNK3 showing high score in the local alig nment between JNK3 and BMAL2
<400> 21
   Glu Glu Lys Thr Lys Asn Gly Val Val Lys Gly Gln Pro Ser Pro Ser
   1               5                   10                  15
   Gly Ala Ala Val Asn Ser Ser Glu Ser Leu Pro Pro Ser Ser Ser
               20                  25                  30
<210> 22
   <211> 31
   <212> PRT
   <213> homo sapiens
<220>
   <221> misc_feature
   <223> Partial oligopeptide of BMAL2 showing high score in the local ali gnment between JNK3 and BMAL2
<400> 22
   Asp Asp Ser Ser Pro Thr Gly Leu Met Lys Asp Thr His Thr Val Asn
   1               5                   10                  15
   Cys Arg Ser Met Ser Asn Lys Glu Leu Phe Pro Pro Ser Pro Ser
               20                  25                  30
<210> 23
   <211> 10
   <212> PRT
   <213> homo sapiens
<220>
   <221> misc_feature
   <223> Partial oligopeptide of JNK3 showing high score in the local alig nment between JNK3 and BMAL2
<400> 23
   Glu Gln Leu Gly Thr Pro Cys Pro Glu Phe
   1               5                   10
<210> 24
   <211> 10
   <212> PRT
   <213> homo sapiens
<220>
   <221> misc_feature
   <223> Partial oligopeptide of BMAL2 showing high score in the local ali gnment between JNK3 and BMAL2
<400> 24
   Glu Leu Leu Gly Thr Ser Cys Tyr Glu Tyr
   1               5                   10
<210> 25
   <211> 23
   <212> PRT
   <213> homo sapiens
<220>
   <221> misc_feature
   <223> Partial oligopeptide of JNK3 showing high score in the local alig nment between JNK3 and BMAL2
<400> 25
   Ser Lys Ser Lys Val Asp Asn Gln Phe Tyr Ser Val Glu Val Gly Asp
   1               5                   10                  15
   Ser Thr Phe Thr Val Leu Lys
               20
<210> 26
   <211> 23
   <212> PRT
   <213> homo sapiens
<220>
   <221> misc_feature
   <223> Partial oligopeptide of BMAL2 showing high score in the local ali gnment between JNK3 and BMAL2
<400> 26
   Ser Lys Glu Lys Ile Leu Thr Asp Ser Tyr Lys Phe Arg Ala Lys Asp
   1               5                   10                  15
   Gly Ser Phe Val Thr Leu Lys
               20
<210> 27
   <211> 12
   <212> PRT
   <213> homo sapiens
<220>
   <221> misc_feature
   <223> Partial oligopeptide of JNK3 showing high score in the local alig nment between JNK3 and BMAL2
<400> 27
   Ser Lys Ser Lys Val Asp Asn Gln Phe Tyr Ser Val
   1               5                   10
<210> 28
   <211> 12
   <212> PRT
   <213> homo sapiens
<220>
   <221> misc_feature
   <223> Partial oligopeptide of BMAL2 showing high score in the local ali gnment between JNK3 and BMAL2
<400> 28
   Ser Lys Lys Lys Glu His Arg Lys Phe Tyr Thr Ile
   1               5                   10